(19) Europäisches Patentamt
European Patent Office
Office européen des brevets

(11) EP 2 471 777 A1

(12) # EUROPEAN PATENT APPLICATION
published in accordance with Art. 153(4) EPC

(43) Date of publication:
**04.07.2012 Bulletin 2012/27**

(21) Application number: **10811981.9**

(22) Date of filing: **27.08.2010**

(51) Int Cl.:
***C07D 217/06*** *(2006.01)* ***A61K 31/472*** *(2006.01)*
***A61P 1/16*** *(2006.01)* ***A61P 3/04*** *(2006.01)*
***A61P 3/06*** *(2006.01)* ***A61P 3/10*** *(2006.01)*
***A61P 9/10*** *(2006.01)* ***A61P 15/08*** *(2006.01)*
***A61P 27/02*** *(2006.01)* ***A61P 43/00*** *(2006.01)*

(86) International application number:
**PCT/JP2010/064550**

(87) International publication number:
**WO 2011/024932 (03.03.2011 Gazette 2011/09)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **28.08.2009 JP 2009198540**

(71) Applicant: **Daiichi Sankyo Company, Limited**
**Toyko 103-8426 (JP)**

(72) Inventors:
- **UTO, Yoshikazu**
  **Shinagawa-ku**
  **Tokyo 140-8710 (JP)**
- **KARASAWA, Hiroshi**
  **Shinagawa-ku**
  **Tokyo 140-8710 (JP)**
- **TAKAISHI, Kiyosumi**
  **Shinagawa-ku**
  **Tokyo 140-8710 (JP)**

(74) Representative: **Wallace, Sheila Jane et al**
**Marks & Clerk LLP**
**90 Long Acre**
**London**
**WC2E 9RA (GB)**

(54) **NOVEL TETRAHYDROISOQUINOLINE COMPOUNDS**

(57) The present invention relates to a compound or a pharmacologically acceptable salt thereof having an excellent DGAT inhibitory effect and feeding suppressant effect. The present invention provides trans-6-[3-(2,4-difluoro-phenyl)-ureido]-3,4-dihydro-1H-isoquinoline-2-carboxylic acid 4-carboxymethyl-cyclohexyl ester, trans-6-[3-(2-chloro-phenyl)-ureido]-3,4-dihydro-1H-isoquinoline-2-carboxylic acid 4-carboxymethyl-cyclohexyl ester, trans-6-[3-(2,3-difluoro-phenyl)-ureido]-3,4-dihydro-1H-isoquinoline-2-carboxylic acid 4-carboxymethyl-cyclohexyl ester, trans-6-[3-(2,5-difluoro-phenyl)-ureido]-3,4-dihydro-1H-isoquinoline-2-carboxylic acid 4-carboxymethyl-cyclohexyl ester, trans-6-[3-(2,6-difluoro-phenyl)-ureido]-3,4-dihydro-1H-isoquinoline-2-carboxylic acid 4-carboxymethyl-cyclohexyl ester, or the like, or a pharmacologically acceptable salt thereof.



Printed by Jouve, 75001 PARIS (FR)

## Description

### Technical Field

The present invention relates to a compound or a pharmacologically acceptable salt thereof having a particular chemical structure having an excellent acyl-coenzyme A: diacylglycerol acyltransferase (Acyl-CoA: diacylglycerol acyltransferase, hereinafter, also referred to as DGAT) inhibitory effect and an excellent feeding suppressant effect.

### Background Art

Adiposity is a condition of having a significantly greater body weight than normal as a result of accumulation of neutral fats (triacylglycerol or triglyceride, hereinafter also referred to as TG) in fat cells due to continuous excess energy intake compared to energy consumption (Non-Patent Document 1). Adiposity leads to life-style related disease (e.g., hyperlipidemia, hypertriglyceridemia, diabetes, hypertension, and arteriosclerosis), cerebrovascular disorder, coronary artery disease, respiratory abnormality, lower back pain, knee osteoarthritis, gout, cholelithiasis, and so on. Adiposity with a complication of these diseases or adiposity which may later lead to such a complication is defined as obesity and treated as a disease.

Moreover, in recent years, obesity has been shown to be one of the major causes of a life-style related disease called metabolic syndrome (Non-Patent Document 2). It has been reported that in individuals with obesity, fatty acids and factors such as TNN-α are released from accumulated visceral fats, and induce insulin resistance in skeletal muscles, the liver, and fat tissue while facilitating the synthesis of neutral fats in the liver, resulting in hyperlipidemia. Furthermore, an increase in serum insulin concentration induced by the insulin resistance increases peripheral vascular resistance via increased renal reabsorption of Na ions and activation of sympathetic nerves, causing hypertension. Hyperlipidemia, diabetes, and hypertension caused by obesity are also thought to trigger angiopathy such as cerebrovascular disorder or coronary artery disease caused by arteriosclerosis, resulting in severe, life-threatening clinical conditions.

Currently, drug therapy has been practised on obesity based on the provisions of each country, and centrally acting anorectics such as mazindol (Non-Patent Document 3) and sibutramine (Non-Patent Document 4) and lipid absorption inhibitors such as the pancreatic lipase inhibitor orlistat are prescribed mainly for the purpose of controlling calorie intake. These drugs offer low satisfaction with treatment, although achieving some therapeutic effects. The centrally acting anorectics have adverse effects such as dry mouth, constipation, gastric discomfort, and in some cases, auditory hallucination and visual hallucination. Orlistat (Non-Patent Document 5) may cause adverse effects in the gastrointestinal tract, such as diarrhea, incontinence, steatorrhea, and flatus. Accordingly, there is a need for the development of more potent drugs with fewer adverse effects. Under such circumstances, active research and development has been conducted with the aim of developing novel anti-obesity drugs, most of which are anorectics.

Animals and plants store lipids as insoluble TG and produce energy by catabolizing TG according to need. TG taken from food is hydrolyzed into free fatty acid and monoacylglycerol in the lumen of the small intestine by the action of bile acid and pancreatic lipase. Micelles composed of free fatty acid, monoacylglycerol, and bile acid are absorbed into small intestinal epithelial cells in which TG is then re-synthesized in the endoplasmic reticulum by the action of acyl-coenzyme A synthetase (hereinafter, referred to as ACS), acyl-coenzyme A: monoacylglycerol acyltransferase, and DGAT. TG is combined with phospholipid, cholesterol, and apolipoprotein and secreted in the form of chylomicron into the lymph vessels in the stomach and intestine. TG is then secreted into the blood through the lymphatic trunk and transferred to the periphery for use. On the other hand, TG is also synthesized in fat tissue from glycerol 3-phosphate and free fatty acid by the action of ACS, glycerol 3-phosphate acyltransferase, lysophosphatidic acid acyltransferase, and DGAT (Non-Patent Document 6). TG taken excessively is thus accumulated in fat tissue, resulting in obesity.

DGAT, an intracellular enzyme found in the endoplasmic reticulum, catalyzes the most important reaction in the final step in the pathway of TG synthesis, i.e., the reaction of transferring acyl groups of acyl-coenzyme A to the 3 position of 1,2-diacylglycerol (Non-Patent Documents 7 to 9). It has been reported that DGAT has two isozymes DGAT1 (Non-Patent Document 10) and DGAT2 (Non-Patent Document 11). Since DGAT1 and DGAT2 are highly expressed in the small intestine and fat tissue and in the liver and fat tissue, respectively, it is believed that DGAT1 is involved mainly in fat absorption from the small intestine and fat accumulation in fat tissue and DGAT2 is involved mainly in TG synthesis or VLDL (very low density lipoprotein) secretion in the liver and fat accumulation in fat tissue. Although the difference between the roles of DGAT1 and DGAT2 has not yet been fully elucidated, the association of DGAT with obesity, lipid metabolism, glucose metabolism, and the like has been suggested (Non-Patent Document 12). DGAT is a key enzyme of TG synthesis in gastrointestinal epithelial cells and fat tissue. Drugs which inhibit DGAT suppress the TG synthesis and thus suppress fat absorption in the gastrointestinal tract and fat accumulation in fat tissue. Accordingly, such drugs are expected to be useful as therapeutic or preventive agents for, for example, adiposity, obesity, hyperlipidemia, hypertriglyceridemia, lipidosis, insulin resistance syndrome, diabetes, nonalcoholic steatohepatitis, or hyperlipidemia, hypertriglyceridemia, lipidosis, insulin resistance syndrome, diabetes, nonalcoholic steatohepatitis, hypertension, arteriosclerosis, cerebrovascular disorder, or coronary artery disease caused by obesity (Non-Patent Documents 13 to 17).

Anorectics directly or indirectly regulate the system of appetite control, and their mechanisms of action are broadly classified into central and peripheral actions. The centrally acting anorectics directly suppress appetite through their action on the hypothalamic neuronal system containing the feeding and satiety centers or on the monoaminergic neuronal system in the brain regulating this hypothalamic neuronal system. On the other hand, the peripherally acting anorectics indirectly suppress appetite through their action on the mechanism of detecting and transmitting information on nutrition intake from diets or accumulation of excess energy.

In recent years, the mechanism has been increasingly evident, in which, for example, gastrointestinal hormone (CCK, GLP-1, PYY, etc.) (Non-Patent Document 18) secreted in close relation to the digestion and absorption of food, or leptin (Non-Patent Document 19) secreted from fat cells in response to the amount of energy accumulated (amount of fats) conveys hormonal or neuronal signals regulating appetites from the periphery to the central nervous system. Novel anorectics associated with these peripheral signals are expected to serve as more effective anti-obesity drugs with fewer adverse reactions.

Patent Document 1 discloses compounds structurally similar to compounds of the present invention. This document describes compounds comprising two substituted phenyl groups bound via urea and amide bonds or via urea and ester bonds with a tetrahydroisoquinoline ring, and use thereof as a DGAT inhibitor. Moreover, Patent Document 2 describes use of these compounds as a feeding suppressant. However, these patent documents merely disclose compounds wherein the nitrogen atom on the tetrahydroisoquinoline ring is substituted by a substituted phenyl group via an amide or ester bond. On the other hand, in the compounds of the present invention, the nitrogen atom on the tetrahydroisoquinoline ring is substituted by a 4-carboxymethyl-cyclohexyl group via an ester bond.

Citation List

Patent Documents

Patent Document 1: US 2007/0249620
Patent Document 2: International Publication No. WO2007/074753

Non-Patent Documents

Non-Patent Document 1: Eiji Itagaki, "STEP series, Metabolism, Endocrinology", KAIBASHOBO, LTD. 1st ed., 1998, p. 105 Non-Patent Document 2: Zimmet, P. et al., Nature, 2001, vol. 414, p. 782-787
Non-Patent Document 3: Engstrom, R. G. et al., Arch. Intern. Pharmacodyn., 1975, vol. 214, p. 308-321
Non-Patent Document 4: Bray, G. A. et al., Obes. Res., 1996, vol. 4, p. 263-270
Non-Patent Document 5: Davidson, M. H. et al., The Journal of the American Medical Association, 1999, vol. 281, p. 235-242
Non-Patent Document 6: Coleman, R., Bell, R., J. Biol. Chem., 1976, vol. 251, p. 4537-4543
Non-Patent Document 7: Coleman, R., Methods in Enzymology, 1992, vol. 209, p. 98-104
Non-Patent Document 8: Lehner, R., Kuksis, A., Prog. Lipid Res., 1996, vol. 35, p. 169-201
Non-Patent Document 9: R. Bell., Ann. Rev. Biochem., 1980, vol. 49, p. 459-487
Non-Patent Document 10: Cases, S. et al., Proc. Natl. Acad. Sci. USA., 1998, vol. 95, p. 13018-13023
Non-Patent Document 11: Cases, S. et al., J. Biol. Chem., 2001, vol. 276, p. 38870-38876
Non-Patent Document 12: Coleman, R. A., Lee, D. P., Progress in Lipid Research, 2004, vol. 43, p. 134-176
Non-Patent Document 13: Smith, S. J. et al., Nat. Genet., 2000, vol. 25, p. 87-90
Non-Patent Document 14: Chen, H. C., J. Clin. Invest., 2002, vol. 109, p. 1049-1055
Non-Patent Document 15: Buhman, K. K., J. Biol. Chem., 2002, vol. 277, p. 25474-25479
Non-Patent Document 16: Gaziano, J., et al., Circulation, 1997, vol. 96, p. 2520-2525
Non-Patent Document 17: Yamaguchi, K. et al., Hepatology, 2008, vol. 47, p. 625-635
Non-Patent Document 18: Strader, A. D. et al., Gastroenterology, 2005, vol. 128, p. 175-191
Non-Patent Document 19: Campfield, L. A. et al., Science, 1995, vol. 269, p. 546-549

## Summary of the Invention

### Problems to be Solved by the Invention

The present inventors have conducted diligent studies for compounds having a DGAT inhibitory effect and a feeding suppressant effect and consequently found that compounds having a particular chemical structure have an excellent DGAT inhibitory effect, particularly, a high inhibitory effect against DGAT1. The present inventors have also found that these compounds have an excellent feeding suppressant effect. The present inventors have further found that these compounds are useful as an active ingredient for pharmaceutical agents intended for the prevention and/or treatment of a disease selected from the group consisting of adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, nonalcoholic steatohepatitis, polycystic ovary syndrome, arteriosclerosis, atherosclerosis, diabetic atherosclerosis, ischemic heart disease, and bulimia, or as an active ingredient for pharmaceutical agents intended for the treatment and/or prevention of a disease selected from the group consisting of hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, nonalcoholic steatohepatitis, polycystic ovary syndrome, arteriosclerosis, atherosclerosis, diabetic atherosclerosis, hypertension, cerebrovascular disorder, coronary artery disease, fatty liver, respiratory abnormality, lower back pain, knee osteoarthritis, gout, and cholelithiasis caused by obesity.

Furthermore, the present inventors have found that these compounds are also excellent in terms of high safety, long-lasting effect, low transfer into the central nervous system, high enzymatic selectivity, and little risk of inducing anemia. Based on these findings, the present invention has been completed.

### Means for Solving the Problems

The present invention relates to:

(1) trans-6-[3-(2,4-difluoro-phenyl)-ureido]-3,4-dihydro-1H-isoquinoline-2-carboxylic acid 4-carboxymethyl-cyclohexyl ester,
trans-6-[3-(2-chloro-phenyl)-ureido]-3,4-dihydro-1H-isoquinoline-2-carboxylic acid 4-carboxymethyl-cyclohexyl ester,
trans-6-[3-(2,3-difluoro-phenyl)-ureido]-3,4-dihydro-1H-isoquinoline-2-carboxylic acid 4-carboxymethyl-cyclohexyl ester,
trans-6-[3-(2,5-difluoro-phenyl)-ureido]-3,4-dihydro-1H-isoquinoline-2-carboxylic acid 4-carboxymethyl-cyclohexyl ester,
trans-6-[3-(2,6-difluoro-phenyl)-ureido]-3,4-dihydro-1H-isoquinoline-2-carboxylic acid 4-carboxymethyl-cyclohexyl ester,
trans-6-[3-(2-fluoro-3-methyl-phenyl)-ureido]-3,4-dihydro-1H-isoquinoline-2-carboxylic acid 4-carboxymethyl-cyclohexyl ester,
trans-6-[3-(2-fluoro-5-methyl-phenyl)-ureido]-3,4-dihydro-1H-isoquinoline-2-carboxylic acid 4-carboxymethyl-cyclohexyl ester,
trans-6-[3-(2-fluoro-4-methyl-phenyl)-ureido]-3,4-dihydro--1H-isoquinoline-2-carboxylic acid 4-carboxymethyl-cyclohexyl ester,
trans-6-[3-(2-fluoro-6-methyl-phenyl)-ureido]-3,4-dihydro-1H-isoquinoline-2-carboxylic acid 4-carboxymethyl-cyclohexyl ester,
trans-6-[3-(3-chloro-2-fluoro-phenyl)-ureido]-3,4-dihydro-1H-isoquinoline-2-carboxylic acid 4-carboxymethyl-cyclohexyl ester,
trans-6-[3-(4-chloro-2-fluoro-phenyl)-ureido]-3,4-dihydro-1H-isoquinoline-2-carboxylic acid 4-carboxymethyl-cyclohexyl ester,
trans-6-[3-(5-chloro-2-fluoro-phenyl)-ureido]-3,4-dihydro-1H-isoquinoline-2-carboxylic acid 4-carboxymethyl-cyclohexyl ester,
trans-6-[3-(2-chloro-6-fluoro-phenyl)-ureido]-3,4-dihydro-1H-isoquinoline-2-carboxylic acid 4-carboxymethyl-cyclohexyl ester,
trans-6-[3-(2,3,4-trifluoro-phenyl)-ureido]-3,4-dihydro-1H-isoquinoline-2-carboxylic acid 4-carboxymethyl-cyclohexyl ester,
trans-6-[3-(2,4,6-trifluoro-phenyl)-ureido]-3,4-dihydro-1H-isoquinoline-2-carboxylic acid 4-carboxymethyl-cyclohexyl ester,

trans-6-[3-(2-fluoro-4-trifluoromethyl-phenyl)-ureido]-3,4-dihydro-1H-isoquinoline-2-carboxylic acid 4-carboxymethyl-cyclohexyl ester,
trans-6-[3-(2-trifluoromethyl-phenyl)-ureido]-3,4-dihydro-1H-isoquinoline-2-carboxylic acid 4-carboxymethyl-cyclohexyl ester,
trans-6-[3-(4-fluoro-2-trifluoromethyl-phenyl)-ureido]-3,4-dihydro-1H-isoquinoline-2-carboxylic acid 4-carboxymethyl-cyclohexyl ester,
trans-6-[3-(4-methoxy-2-methyl-phenyl)-ureido]-3,4-dihydro-1H-isoquinoline-2-carboxylic acid 4-carboxymethyl-cyclohexyl ester,
trans-6-[3-(2-chloro-4-methyl-phenyl)-ureido]-3,4-dihydro-1H-isoquinoline-2-carboxylic acid 4-carboxymethyl-cyclohexyl ester,
trans-6-[3-(2-fluoro-3-trifluoromethyl-phenyl)-ureido]-3,4-dihydro-1H-isoquinoline-2-carboxylic acid 4-carboxymethyl-cyclohexyl ester,
trans-6-[3-(2-fluoro-5-trifluoromethyl-phenyl)-ureido]-3,4-dihydro-1H-isoquinoline-2-carboxylic acid 4-carboxymethyl-cyclohexyl ester,
trans-6-[3-(2,4-dimethyl-phenyl)-ureido]-3,4-dihydro-1H-isoquinoline-2-carboxylic acid 4-carboxymethyl-cyclohexyl ester,
trans-6-[3-(2,4-dichloro-phenyl)-ureido]-3,4-dihydro-1H-isoquinoline-2-carboxylic acid 4-carboxymethyl-cyclohexyl ester,
trans-6-[3-(2-fluoro-6-trifluoromethyl-phenyl)-ureido]-3,4-dihydro-1H-isoquinoline-2-carboxylic acid 4-carboxymethyl-cyclohexyl ester,
trans-6-[3-(4-chloro-2-methyl-phenyl)-ureido]-3,4-dihydro-1H-isoquinoline-2-carboxylic acid 4-carboxymethyl-cyclohexyl ester,
trans-6-[3-(2-chloro-3-methyl-phenyl)-ureido]-3,4-dihydro-1H-isoquinoline-2-carboxylic acid 4-carboxymethyl-cyclohexyl ester,
trans-6-[3-(4-chloro-2-trifluoromethyl-phenyl)-ureido]-3,4-dihydro-1H-isoquinoline-2-carboxylic acid 4-carboxymethyl-cyclohexyl ester,
trans-6-[3-(2-chloro-4-trifluoromethyl-phenyl)-ureido]-3,4-dihydro-1H-isoquinoline-2-carboxylic acid 4-carboxymethyl-cyclohexyl ester,
trans-6-[3-(3-fluoro-4-methyl-phenyl)-ureido]-3,4-dihydro-1H-isoquinoline-2-carboxylic acid 4-carboxymethyl-cyclohexyl ester,
trans-6-[3-(3,5-difluoro-phenyl)-ureido]-3,4-dihydro-1H-isoquinoline-2-carboxylic acid 4-carboxymethyl-cyclohexyl ester,
trans-6-[3-(3-chloro-4-fluoro-phenyl)-ureido]-3,4-dihydro-1H-isoquinoline-2-carboxylic acid 4-carboxymethyl-cyclohexyl ester,
trans-6-[3-(3,5-dimethyl-phenyl)-ureido]-3,4-dihydro-1H-isoquinoline-2-carboxylic acid 4-carboxymethyl-cyclohexyl ester,
trans-6-[3-(3-chloro-4-methyl-phenyl)-ureido]-3,4-dihydro-1H-isoquinoline-2-carboxylic acid 4-carboxymethyl-cyclohexyl ester,
trans-6-[3-(2,3-dimethyl-phenyl)-ureido]-3,4-dihydro-1H-isoquinoline-2-carboxylic acid 4-carboxymethyl-cyclohexyl ester,
trans-6-[3-(3-chloro-2-methyl-phenyl)-ureido]-3,4-dihydro-1H-isoquinoline-2-carboxylic acid 4-carboxymethyl-cyclohexyl ester,
trans-6-[3-(2,3-dichloro-phenyl)-ureido]-3,4-dihydro-1H-isoquinoline-2-carboxylic acid 4-carboxymethyl-cyclohexyl ester,
trans-6-[3-(4-fluoro-3-methyl-phenyl)-ureido]-3,4-dihydro-1H-isoquinoline-2-carboxylic acid 4-carboxymethyl-cyclohexyl ester,
trans-6-[3-(3,5-dimethoxy-phenyl)-ureido]-3,4-dihydro-1H-isoquinoline-2-carboxylic acid 4-carboxymethyl-cyclohexyl ester,
trans-6-[3-(3,4-dichloro-phenyl)-ureido]-3,4-dihydro-1H-isoquinoline-2-carboxylic acid 4-carboxymethyl-cyclohexyl ester,
trans-6-[3-(3-chloro-4-methoxy-phenyl)-ureido]-3,4-dihydro-1H-isoquinoline-2-carboxylic acid 4-carboxymethyl-cyclohexyl ester,
trans-6-[3-(2-fluoro-3-methoxy-phenyl)-ureido]-3,4-dihydro-1H-isoquinoline-2-carboxylic acid 4-carboxymethyl-cyclohexyl ester,
trans-6-[3-(3,5-dichloro-phenyl)-ureido]-3,4-dihydro-1H-isoquinoline-2-carboxylic acid 4-carboxymethyl-cyclohexyl ester,
trans-6-[3-(3,4-dimethoxy-phenyl)-ureido]-3,4-dihydro-1H-isoquinoline-2-carboxylic acid 4-carboxymethyl-cyclohexyl ester,

trans-6-[3-(3-fluoro-4-methoxy-phenyl)-ureido]-3,4-dihydro-1H-isoquinoline-2-carboxylic acid 4-carboxymethyl-cyclohexyl ester, or

trans-6-[3-(3-chloro-phenyl)-ureido]-3,4-dihydro-1H-isoquinoline-2-carboxylic acid 4-carboxymethyl-cyclohexyl ester,

or a pharmacologically acceptable salt thereof.

The present invention also relates to:

(2) An acyl-coenzyme A: diacylglycerol acyltransferase inhibitor comprising as an active ingredient thereof a compound or pharmacologically acceptable salt thereof according to (1);

(3) A feeding suppressant and/or an anorectic comprising as an active ingredient thereof a compound or pharmacologically acceptable salt thereof according to (1);

(4) A pharmaceutical composition comprising as an active ingredient thereof a compound or pharmacologically acceptable salt thereof according to (1);

(5) The pharmaceutical composition according to (4), wherein the pharmaceutical composition has an acyl-coenzyme A: diacylglycerol acyltransferase inhibitory effect;

(6) The pharmaceutical composition according to (4), wherein the pharmaceutical composition has a feeding suppressant effect and/or an anorectic effect;

(7) The pharmaceutical composition according to (4), wherein the pharmaceutical composition is intended for the treatment and/or prevention of a disease which is treated and/or prevented by an acyl-coenzyme A: diacylglycerol acyltransferase inhibitory effect;

(8) The pharmaceutical composition according to (4), wherein the pharmaceutical composition is intended for the treatment and/or prevention of a disease caused by increased acyl-coenzyme A: diacylglycerol acyltransferase activity;

(9) The pharmaceutical composition according to (4), wherein the pharmaceutical composition is intended for the treatment and/or prevention of a disease whose symptoms are treated, improved, alleviated and/or prevented by inhibiting acyl-coenzyme A: diacylglycerol acyltransferase such that triglyceride synthesis is inhibited, resulting in suppressed absorption of triglyceride;

(10) The pharmaceutical composition according to (4), wherein the pharmaceutical composition is intended for the treatment and/or prevention of a disease whose symptoms are treated, improved, alleviated and/or prevented by inhibiting acyl-coenzyme A: diacylglycerol acyltransferase such that triglyceride synthesis is inhibited

(11) The pharmaceutical composition according to (4), wherein the pharmaceutical composition is intended for the treatment and/or prevention of adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, nonalcoholic steatohepatitis, polycystic ovary syndrome, arteriosclerosis, atherosclerosis, diabetic atherosclerosis, ischemic heart disease, or bulimia;

(12) The pharmaceutical composition according to (4), wherein the pharmaceutical composition is intended for the treatment and/or prevention of adiposity or obesity;

(13) The pharmaceutical composition according to (4), wherein the pharmaceutical composition is intended for the treatment and/or prevention of hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, nonalcoholic steatohepatitis, polycystic ovary syndrome, arteriosclerosis, atherosclerosis, diabetic atherosclerosis, hypertension, cerebrovascular disorder, coronary artery disease, fatty liver, respiratory abnormality, lower back pain, knee osteoarthritis, gout, or cholelithiasis caused by obesity;

(14) The pharmaceutical composition according to (4), wherein the pharmaceutical composition is intended for the treatment and/or prevention of hyperlipidemia, hypertriglyceridemia, diabetes, arteriosclerosis, or hypertension caused by obesity;

(15) The pharmaceutical composition according to (4), wherein the pharmaceutical composition is intended for the treatment and/or prevention of diabetes;

(16) The pharmaceutical composition according to (4), wherein the pharmaceutical composition is intended for the suppression of fat absorption from the small intestine;

(17) Use of a compound or pharmacologically acceptable salt thereof according to (1) for producing a pharmaceutical composition;

(18) The use according to (17), wherein the pharmaceutical composition is a composition intended for the inhibition of acyl-coenzyme A: diacylglycerol acyltransferase;

(19) The use according to (17), wherein the pharmaceutical composition is a composition intended for the suppression

of feeding and/or appetite;

(20) The use according to (17), wherein the pharmaceutical composition is a composition intended for the treatment and/or prevention of adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, nonalcoholic steatohepatitis, polycystic ovary syndrome, arteriosclerosis, atherosclerosis, diabetic atherosclerosis, ischemic heart disease, or bulimia;

(21) The use according to (17), wherein the pharmaceutical composition is a composition intended for the treatment and/or prevention of adiposity or obesity;

(22) The use according to (17), wherein the pharmaceutical composition is a composition intended for the treatment and/or prevention of hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, nonalcoholic steatohepatitis, polycystic ovary syndrome, arteriosclerosis, atherosclerosis, diabetic atherosclerosis, hypertension, cerebrovascular disorder, coronary artery disease, fatty liver, respiratory abnormality, lower back pain, knee osteoarthritis, gout, or cholelithiasis caused by obesity;

(23) The use according to (17), wherein the pharmaceutical composition is a composition intended for the treatment and/or prevention of hyperlipidemia, hypertriglyceridemia, diabetes, arteriosclerosis, or hypertension caused by obesity;

(24) The use according to (17), wherein the pharmaceutical composition is a composition intended for the treatment and/or prevention of diabetes;

(25) The use according to (17), wherein the pharmaceutical composition is a composition intended for the suppression of fat absorption from the small intestine;

(26) A method for inhibiting acyl-coenzyme A: diacylglycerol acyltransferase, comprising administering a pharmacologically effective amount of a compound or pharmacologically acceptable salt thereof according to (1) to a warm-blooded animal;

(27) A method for suppressing feeding and/or appetite, comprising administering a pharmacologically effective amount of a compound or pharmacologically acceptable salt thereof according to (1) to a warm-blooded animal;

(28) A method for treating and/or preventing a disease, comprising administering a pharmacologically effective amount of a compound or pharmacologically acceptable salt thereof according to (1) to a warm-blooded animal;

(29) The method according to (28), wherein the disease is adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, nonalcoholic steatohepatitis, polycystic ovary syndrome, arteriosclerosis, atherosclerosis, diabetic atherosclerosis, ischemic heart disease, or bulimia;

(30) The method according to (28), wherein the disease is adiposity or obesity;

(31) The method according to (28), wherein the disease is hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, nonalcoholic steatohepatitis, polycystic ovary syndrome, arteriosclerosis, atherosclerosis, diabetic atherosclerosis, hypertension, cerebrovascular disorder, coronary artery disease, fatty liver, respiratory abnormality, lower back pain, knee osteoarthritis, gout, or cholelithiasis caused by obesity;

(32) The method according to (28), wherein the disease is hyperlipidemia, hypertriglyceridemia, diabetes, arteriosclerosis, or hypertension caused by obesity;

(33) The method according to (28), wherein the disease is diabetes;

(34) A method for suppressing fat absorption from the small intestine, comprising administering a pharmacologically effective amount of a compound or pharmacologically acceptable salt thereof according to (1) to a warm-blooded animal; and

(35) The method according to any one of (26) to (34), wherein the warm-blooded animal is a human.

A compound of the present invention or a pharmacologically acceptable salt thereof may also contain, at a non-natural ratio, atomic isotope(s) of one or more of the atoms constituting such a compound. Examples of the atomic isotope(s) include deuterium ($^2H$), tritium ($^3H$), iodine-125 ($^{125}I$), and carbon-14 ($^{14}C$). Moreover, a compound may be radiolabeled with a radioisotope, for example, tritium ($^3H$), iodine-125 ($^{125}I$), or carbon-14 ($^{14}C$). Such radiolabeled compounds are useful as therapeutic or preventive agents, research reagents, for example, assay reagents, and diagnostic agents, for example, *in vivo* image diagnostic agents. All isotopic variants of the compounds of the present invention are included in the scope of the present invention, regardless of whether or not they are radioactive.

A "pharmacologically acceptable salt thereof" refers to a salt that is free of prominent toxicity and which can

be used as a pharmaceutical. A compound of the present invention can be converted to a salt by reacting with a base because a compound of the present invention has an acidic group such as a carboxyl group.

Examples of salts based on acidic groups include metal salts such as alkali metal salts such as sodium salts, potassium salts or lithium salts, alkaline earth metal salts such as calcium salts or magnesium salts, metal salts such as aluminium salts or iron salts; amine salts such as inorganic salts such as ammonium salts, or organic salts such as salts of t-octylamine, dibenzylamine, morpholine, glucosamine, phenylglycine alkyl esters, ethylenediamine, N-methylglucamine, guanidine, diethylamine, triethylamine, dicyclohexylamine, N,N'-dibenzylethylenediamine, chloroprocaine, procaine, diethanolamine, N-benzylphenethylamine, piperazine, tetramethylammonium or tris(hydroxymethyl)aminomethane; and, salts of amino acids such as salts of glycine, lysine, arginine, ornithine, glutamic acid and aspartic acid.

A compound of the present invention or a pharmacologically acceptable salt thereof may become a hydrate by incorporating water molecule(s) by being left in the atmosphere or by recrystallizing, and such hydrates are also included in the salts of the present invention.

A compound of the present invention or a pharmacologically acceptable salt thereof may become a solvate by absorbing another type of solvent, and such solvates are also included in the salts of the present invention.

## Advantages of the Invention

A compound of the present invention or a pharmacologically acceptable salt thereof has an excellent DGAT inhibitory effect and feeding suppressant effect and is thus useful as a pharmaceutical agent intended for the prevention and/or treatment of a disease selected from the group consisting of adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, nonalcoholic steatohepatitis, polycystic ovary syndrome, arteriosclerosis, atherosclerosis, diabetic atherosclerosis, ischemic heart disease, and bulimia, or a disease selected from the group consisting of hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, nonalcoholic steatohepatitis, polycystic ovary syndrome, arteriosclerosis, atherosclerosis, diabetic atherosclerosis, hypertension, cerebrovascular disorder, coronary artery disease, fatty liver, respiratory abnormality, lower back pain, knee osteoarthritis, gout, and cholelithiasis caused by obesity, in warm-blooded animals (preferably mammals, including humans). Moreover, a compound provided by the present invention or a pharmacologically acceptable salt thereof has an excellent DGAT inhibitory effect and is thus useful as an active ingredient for a pharmaceutical agent intended for the prevention and/or treatment of any of the above diseases in warm-blooded animals (preferably mammals, including humans). Preferably, a compound of the present invention or a pharmacologically acceptable salt thereof can be used as a pharmaceutical agent intended for the treatment of any of the diseases described above.

## Mode for Carrying Out the Invention

A compound or pharmacologically acceptable salt thereof of the present invention can be administered in various forms. Examples of the route of administration include oral administration using tablets, capsules, granules, emulsions, pills, powders, syrups (solutions), and the like and parenteral administration using injections (intravenous, intramuscular, subcutaneous, or intraperitoneal administration), drip infusions, suppositories (rectal administration), and the like. These various formulations can be prepared as drug products according to usual methods using aids usually used in the field of drug formulation such as excipients, binders, disintegrants, lubricants, flavoring agents, dissolving aids, suspending agents, and coating agents in addition to the active ingredient.

In the use as a tablet, examples of carriers that can be used include excipients such as lactose, sucrose, sodium chloride, glucose, urea, starch, calcium carbonate, kaolin, crystalline cellulose, and silicic acid; binders such as water, ethanol, propanol, simple syrup, glucose solution, starch solution, gelatin solution, carboxymethylcellulose, shellac, methylcellulose, potassium phosphate, and polyvinylpyrrolidone; disintegrants such as dry starch, sodium alginate, agar powder, laminaran powder, sodium hydrogencarbonate, calcium carbonate, polyoxyethylene sorbitan fatty acid esters, sodium lauryl sulfate, stearic monoglyceride, starch, and lactose; disintegration inhibitors such as sucrose, stearin, cocoa butter, and hydrogenated oil; absorption enhancers such as quaternary ammonium salts and sodium lauryl sulfate; humectants such as glycerine and starch; adsorbents such as starch, lactose, kaolin, bentonite, and colloidal silicic acid; lubricants such as purified talc, stearate, fluoboric acid powder, and polyethylene glycol, and so forth. Furthermore, tablets coated in usual ways such as, for example, sugar-coated tablets, gelatin-coated tablets, enteric-coated tablets, film-coated tablets, double-layer tablets, and multilayered tablets can be prepared as required.

In the use as a pill, examples of carriers that can be used include excipients such as glucose, lactose, cocoa butter, starch, hydrogenated vegetable oil, kaolin, and talc; binders such as powdered gum arabic, powdered tragacanth,

gelatin, and ethanol; disintegrants such as laminaran and agar, and so forth.

In the use as a suppository, a wide range of carriers known in this field can be used, and examples thereof include polyethylene glycol, cocoa butter, higher alcohols, higher alcohol esters, gelatin, semisynthetic glycerides, and so forth.

In the use as an injection, the formulations can be prepared as solutions, emulsions, or suspensions. Preferably, these solutions, emulsions, and suspensions are sterilized and are isotonic with blood. Solvents for producing these solutions, emulsions, and suspensions are not particularly limited so long as they can be used as diluents for medical use, and examples thereof include water, ethanol, propylene glycol, ethoxylated isostearyl alcohol, polyoxylated isostearyl alcohol, polyoxy ethylene sorbitan fatty acid esters, and so forth. In this case, a sufficient amount of sodium chloride, glucose, or glycerine may be added to the formulation to prepare an isotonic solution, and usual dissolving aids, buffers, soothing agents, and the like may also be added.

Furthermore, coloring agents, preservatives, perfumes, flavoring agents, sweeteners, and the like can be added to the above-mentioned formulations, if necessary. Furthermore, other drugs can also be added.

The amount of active ingredient compound contained in the above-mentioned formulations is not particularly limited, but is usually 0.5 to 70% by weight of the total composition, preferably 1 to 30% by weight.

The dose varies depending on symptoms, age, and the like of the patient (a warm-blooded animal, in particular, a human). In the case of oral administration, the recommended adult daily dosage is from 0.1 mg as the lower limit (preferably 1 mg, more preferably 10 mg) to 2000 mg as the upper limit (preferably 100 mg), which is divided into 1 to 6 doses depending on symptoms.

## Examples

Hereinafter, the present invention will be described more specifically with reference to Examples and Test Examples. However, the scope of the present invention is not intended to be limited to these.

In these Examples, elution in column chromatography was performed under observation by TLC (Thin Layer Chromatography). In the TLC observation, silica gel $60F_{254}$ manufactured by Merck was adopted as the TLC plate; solvents used as eluting solvents in column chromatography were adopted as developing solvents; and a UV detector was adopted as the detection method. Silica gel SK-85 (230-400 mesh) also manufactured by Merck or Chromatorex NH (200-350 mesh, FUJI SILYSIA CHEMICAL LTD.) was used as silica gel for columns. In addition to usual column chromatography, Biotage automatic chromatography apparatus (SP-1) was appropriately used. The solvents described in each Example were used as eluting solvents at the prescribed ratio (or this ratio was changed appropriately according to need). Abbreviations used in Examples mean the following:
mg: milligram, g: gram, mL: milliliter, MHz: megahertz.

In the Examples below, nuclear magnetic resonance (hereinafter, referred to as $^1H$ NMR) spectra were indicated in δ values (ppm) in terms of chemical shift values with tetramethylsilane as standards. Splitting patterns were represented by s for singlet, d for doublet, t for triplet, q for quartet, m for multiplet, and br for broad.

Mass spectrometry (hereinafter, referred to as MS) was conducted by the EI (Electron Ionization), or ESI (Electron Spray Ionization) method.

(Example 1)

trans-6-[3-(2,4-difluoro-phenyl)-ureido]-3,4-dihydro-1H-isoquinoline-2-carboxylic acid 4-carboxymethyl-cyclohexyl ester

[Formula 1]

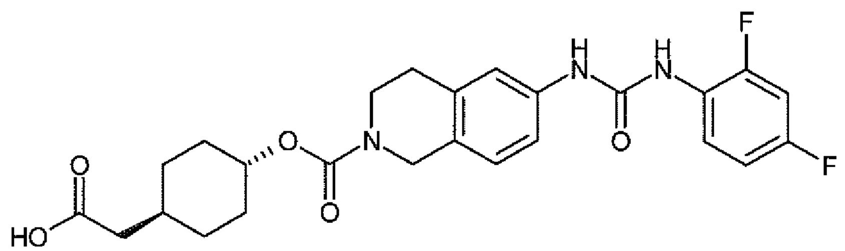

(1a) (1,4-dioxa-spiro[4.5]dec-8-ylidene)-acetic acid methyl ester

To a DMF (50 mL) solution of trimethyl phosphonoacetate (26 mL), sodium hydride (purity: 55% or higher, 7.03 g) was added in small portions at 0°C. The reaction mixture was warmed to room temperature and stirred for 30 minutes. A DMF (50 mL) solution of 1,4-cyclohexanedione monoethylene ketal (25.2 g) was added thereto in small portions at room temperature. This suspension was stirred for 19 hours and diluted with a saturated aqueous solution of ammonium chloride, followed by two extractions with ethyl acetate. The organic layer was washed with saturated brine, then dried over sodium sulfate, and then concentrated. The residue was purified by chromatography (hexane/ethyl acetate=5:1) to obtain the title compound (29.7 g, 87%) as a colorless oil.
$^1$H NMR (400 MHz, $CDCl_3$): δ (ppm) = 5.75-5.65 (1H, m), 3.99 (4H, s), 3.70 (3H, s), 3.01 (2H, t, J = 6.7 Hz), 2.39 (2H, t, J = 6.7 Hz), 1.81-1.75 (4H, m)
MS (EI) m/z: 212 $(M)^+$.

(1b) (1,4-dioxa-spiro[4.5]dec-8-yl)-acetic acid methyl ester

An ethanol (50 mL) suspension of the compound (6.57 g) obtained in Example (1a) and palladium carbon (10% by weight) was hydrogen-reduced at room temperature for 24 hours. The reaction mixture was filtered and concentrated. The residue was purified by chromatography (hexane/ethyl acetate=5:1) to obtain the title compound (4.99 g, 75%) as a colorless oil.
$^1$H NMR (400 MHz, $CDCl_3$): δ (ppm) = 3.94 (4H, s), 3.67 (3H, s), 2.25 (2H, d, J = 7.0 Hz), 1.89-1.81 (1H, m), 1.74 (4H, d, J = 9.8 Hz), 1.63-1.53 (2H, m), 1.36-1.25 (2H, m)
MS (EI) m/z: 214 $(M)^+$.

(1c) (4-oxo-cyclohexyl)-acetic acid methyl ester

A 1 N aqueous hydrochloric acid solution (50 mL)/acetone (200 ml) mixture of the compound (4.99 g) obtained in Example (1b) was stirred at room temperature for 15 hours. The organic solvent was removed using an evaporator. The remaining aqueous solution was subjected to two extractions with ethyl acetate. The organic layer was washed with saturated brine, then dried over sodium sulfate, and then concentrated to obtain the title compound (4.30 g, quantitative yield) as a colorless oil.
$^1$H NMR (400 MHz, $CDCl_3$) δ (ppm) = 3.71 (3H, s), 2.41-2.24 (7H, m), 2.12-2.05 (2H, m), 1.54-1.43 (2H, m);
MS (EI) m/z: 170 $(M)^+$.

(1d) trans-(4-hydroxy-cyclohexyl)-acetic acid methyl ester

To a methanol (50 mL) solution of the compound (4.18 g) obtained in Example (1c), sodium borohydride (1.86 g) was added in small portions at 0°C. The reaction mixture was stirred for 1 hour and diluted with a 10% aqueous ammonium chloride solution (100 mL). The organic solvent was removed using an evaporator. The remaining aqueous solution was subjected to two extractions with ethyl acetate. The organic layer was washed with saturated brine, then dried over sodium sulfate, and then concentrated to obtain trans-(4-hydroxycyclohexyl)-acetic acid methyl ester (2.56 g, 60%) as a colorless oil.
$^1$H NMR (400 MHz, $CDCl_3$): δ (ppm) = 3.67 (3H, s), 3.67-3.60 (1H, m), 2.20 (2H, d, J = 6.7 Hz), 2.00-1.94 (2H, m), 1.82-1.67 (4H, m), 1.35-1.26 (2H, m), 1.10-1.00 (2H, m).

(1e) 6-nitro-3,4-dihydro-1H-isoquinoline-2-carboxylic acid 4-methoxycarbonylmethyl-cyclohexyl ester

To a dichloromethane (100 mL) solution of triphosgene (13.1 g), a dichloromethane (200 mL) solution of the compound (18.9 g) obtained in Example (1d) and pyridine (8.9 mL) was gradually added dropwise at 0°C. The reaction mixture was warmed to room temperature, then stirred for 2 hours, and then concentrated. The residue was vigorously stirred in diisopropyl ether and filtered. The filtrate was concentrated to obtain chloroformate as a colorless oil. A dichloromethane solution (100 mL) of this oil was added at room temperature to a dichloromethane (200 mL) suspension of 6-nitro-1,2,3,4-tetrahydroisoquinoline hydrochloride (J. Med. Chem. 1996, 39, 1084; or J. Org. Chem. 1998, 63, 4116) (21.5 g) and triethylamine (42 mL). After 3.5 hours, the reaction mixture was washed with a saturated aqueous solution of ammonium chloride, then dried (sodium sulfate), and then concentrated. The residue was dissolved in dichloromethane, and hexane was added to the solution until a solid was deposited. The suspension obtained was vigorously stirred, and the solid was collected by filtration and dried to obtain the title compound (30.4 g, 81%) as a pale yellow solid.
$^1$H NMR (400 MHz, $CDCl_3$): δ (ppm)=8.07-8.05 (2H, m), 7.29-7.27 (1H, m), 4.70 (2H, s), 4.67-4.61 (1H, m), 3.73 (2H, t, J=5.2 Hz), 3.68 (3H, s), 2.95 (2H, t, J=5.5 Hz), 2.23 (2H, d, J=6.7 Hz), 2.07-2.03 (2H, m), 1.85-1.76 (3H, m), 1.47-1.37

(2H, m), 1.18-1,08 (2H, m).

(1f) 6-amino-3,4-dihydro-1H-isoquinoline-2-carboxylic acid 4-methoxycarbonylmethyl-cyclohexyl ester

An ethyl acetate (400 mL) suspension of the compound (43.1 g) obtained in Example (1e) and palladium carbon (10%, 4.30 g) was stirred at room temperature for 8 hours in a hydrogen atmosphere. The reaction mixture was filtered through Celite and concentrated. The residue was purified by chromatography (NH silica gel, dichloromethane) to obtain the title compound (39.6 g, quantitative yield).
$^1$H NMR (400 MHz, $CDCl_3$): δ (ppm)=6.91 (1H, d, J=7.4 Hz), 6.55 (1H, dd, J=7.8 and 2.3 Hz), 6.48 (1H, s), 5.31-5.28 (4H, m), 4.65-4.57 (1H, m), 4.49 (2H, s), 3.67 (3H, s), 3.64 (2H, brs), 2.74 (2H, brs), 2.22 (2H, d, J=6.7 Hz), 2.05-2.02 (2H, m), 1.83-1.75 (3H, m), 1.45-1.35 (2H, m), 1.17-1.07 (2H, m).

(1g) trans-6-[3-(2,4-difluoro-phenyl)-ureido]-3,4-dihydro-1H-isoquinoline-2-carboxylic acid 4-carboxymethyl-cyclohexyl ester

A tetrahydrofuran (4 mL) solution of the compound (253 mg) obtained in Example (1f) and 2,4-difluoro-phenyl isocyanate (0.10 mL) was stirred at room temperature for 40 minutes and then concentrated. The residue was purified by chromatography (dichloromethane:ethyl acetate=1:0→4:1) to obtain methyl ester (302 mg, 83%). To a tetrahydrofuran (2 mL)/methanol (2 mL) mixed solution of this methyl ester (302 mg), an aqueous sodium hydroxide solution (1.0 mol/L aqueous solution, 1.2 mL) was added at room temperature. The reaction mixture was stirred for 18 hours and concentrated. The residue was diluted with water, then neutralized with a 1 N aqueous hydrochloric acid solution (1.5 mL), and collected by filtration to obtain the title compound (271 mg, 93%) as a white solid.
$^1$H NMR (400 MHz, DMSO-d6): δ (ppm)=12.0 (1H, s), 8.95 (1H, s), 8.48 (1H, d, J=2.3 Hz), 8.12-8.06 (1H, m), 7.34-7.28 (2H, m), 7.22 (1H, d, J=9.4 Hz), 7.10 (1H, d, J=8.2 Hz), 7.07-7.02 (1H, m), 4.51-4.46 (3H, m), 3.57 (2H, t, J=5.9 Hz), 2.76 (2H, t, J=6.1 Hz), 2.12 (2H, d, J=7.1 Hz), 1.95-1.91 (2H, m), 1.77-1.73 (2H, m), 1.68-1.62 (1H, m), 1.39-1.29 (2H, m), 1.12-1.02 (2H, m)
MS (ESI) m/z: 488 $(M + H)^+$.

(Example 2)

trans-6-[3-(2-chloro-phenyl)-ureido]-3,4-dihydro-1H-isoquinoline-2-carboxylic acid 4-carboxymethyl-cyclohexyl ester

[Formula 2]

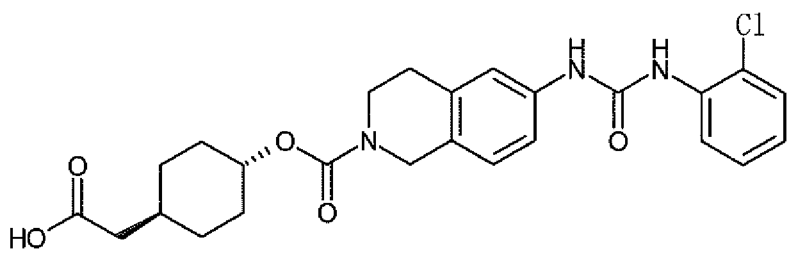

Methyl ester (296 mg, 94%) was obtained in the same way as in Example (1g) from the compound (219 mg) obtained in Example (1f) and 2-chloro-phenyl isocyanate (0.09 mL). This methyl ester (296 mg) was hydrolyzed to obtain the title compound (272 mg, 95%) as a white solid.
$^1$H NMR (400 MHz, DMSO-d6): δ (ppm)=12.1 (1H, s), 9.36 (1H, s), 8.28 (1H, s), 8.17 (1H, dd, J=8.4 and 1.8 Hz), 7.46 (1H, dd, J=7.8 and 1.6 Hz), 7.34 (1H, s), 7.32-7.28 (1H, m), 7.24-7.22 (1H, m), 7.23 (1H, d, J=10.2 Hz), 7.05-7.01 (1H, m), 4.51-4.47 (3H, m), 3.57 (2H, t, J=5.9 Hz), 2.76 (2H, t, J=5.9 Hz), 2.12 (2H, d, J=6.7 Hz), 1.95-1.91 (2H, m), 1.78-1.73 (2H, m), 1.68-1.62 (1H, m), 1.40-1.29 (2H, m), 1.12-1.02 (2H, m)
MS (ESI) m/z: 486 $(M + H)^+$.
(Example 3)

trans-6-[3-(2,3-difluoro-phenyl)-ureido]-3,4-dihydro-1H-isoquinoline-2-carboxylic acid 4-carboxymethyl-cyclohexyl ester

[Formula 3]

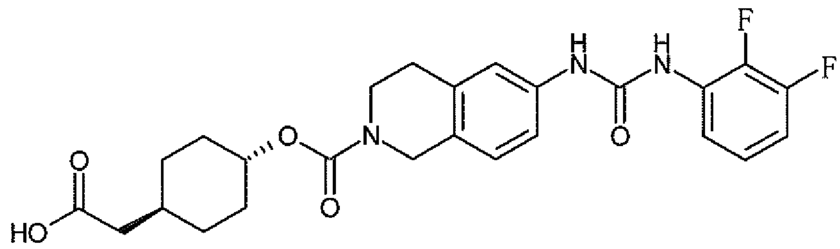

To a dichloromethane (4 mL) solution of triphosgene (82 mg), a dichloromethane solution of the compound (240 mg) obtained in Example (1f) and triethylamine (0.2 mL) was gradually added at room temperature. After 10 minutes, a dichloromethane solution (4 mL) of 2,3-difluoro-aniline (0.07 mL) and triethylamine (0.2 mL) was added thereto. The reaction mixture was stirred for 40 minutes and concentrated. The residue was purified by chromatography (dichloromethane:ethyl acetate=1:0→4:1) to obtain methyl ester (190 mg, 55%).. To a tetrahydrofuran (4 mL)/methanol (4 mL) mixed solution of this methyl ester (190 mg), an aqueous sodium hydroxide solution (1.0 mol/L aqueous solution, 1.4 mL) was added at room temperature. The reaction mixture was stirred for 18 hours and concentrated. The residue was diluted with water, then neutralized with a 1 N aqueous hydrochloric acid solution (1.6 mL), and collected by filtration to obtain the title compound (154 mg, 83%) as an off-white solid.
$^1$H NMR (400 MHz, DMSO-d6): δ (ppm)=12.0 (1H, s), 9.04 (1H, s), 8.72 (1H, d, J=1.9 Hz), 7.96 (1H, dd, J=7.6 and 7.6 Hz), 7.32 (1H, s), 7.22 (1H, d, J=9.8 Hz), 7.17-7.10 (2H, m), 7.06-6.99 (1H, m), 4.51-4.47 (3H, m), 3.57 (2H, t, J=5.6 Hz), 2.76 (2H, t, J=5.9 Hz), 2.12 (2H, d, J=7.1 Hz), 1.95-1.91 (2H, m), 1.78-1.73 (2H, m), 1.68-1.62 (1H, m), 1.40-1.29 (2H, m), 1.12-1.02 (2H, m)
MS (ESI) m/z: 488 $(M + H)^+$.

(Example 4)

trans-6-[3-(2,5-difluoro-phenyl)-ureido]-3,4-dihydro-1H-isoquinoline-2-carboxylic acid 4-carboxymethyl-cyclohexyl ester

[Formula 4]

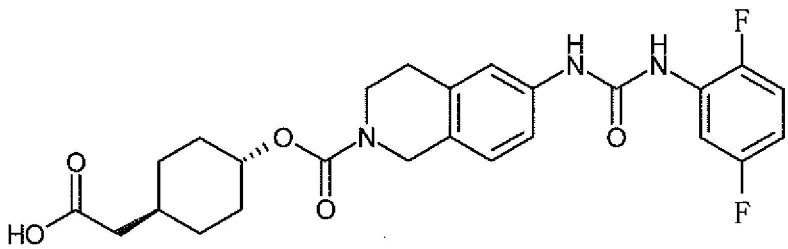

Methyl ester (163 mg, 44%) was obtained in the same way as in Example 3 from the compound (254 mg) obtained in Example (1f) and 2,5-difluoro-aniline (0.22 mL). This methyl ester (163 mg) was hydrolyzed to obtain the title compound (138 mg, 87%) as a white solid.
$^1$H NMR (400 MHz, DMSO-d6): δ (ppm)=12.0 (1H, s), 9.08 (1H, s), 8.74 (1H, s), 8.07-8.02 (1H, m), 7.33 (1H, s), 7.31-7.26 (1H, m), 7.22 (1H, d, J=8.6 Hz), 7.12 (1H, d, J=8.6 Hz), 6.85-6.79 (1H, m), 4.52-4.47 (3H, m), 3.57 (2H, t, J=5.9 Hz), 2.77 (2H, t, J=5.7 Hz), 2.12 (2H, d, J=7.1 Hz), 1.95-1.91 (2H, m), 1.77-1.74 (2H, m), 1.68-1.62 (1H, m), 1.39-1.30 (2H,

m), 1.12-1.02 (2H, m)
MS (ESI) m/z: 488 (M + H)+.

(Example 5)

trans-6-[3-(2,6-difluoro-phenyl)-ureido]-3,4-dihydro-1H-isoquinoline-2-carboxylic acid 4-carboxymethyl-cyclohexyl ester

[Formula 5]

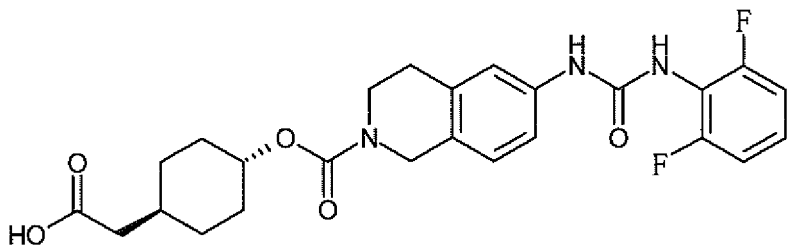

Methyl ester (187 mg, 42%) was obtained in the same way as in Example 3 from the compound (308 mg) obtained in Example (1f) and 2,6-difluoro-aniline (115 mg). This methyl ester (187 mg) was hydrolyzed to obtain the title compound (166 mg, 92%) as a pale yellow solid.
$^1$H NMR (400 MHz, DMSO-d6): δ (ppm)=12.0 (1H, s), 8.86 (1H, s), 8.08 (1H, s), 7.35-7.27 (2H, m), 7.22 (1H, d, J=9.8 Hz), 7.15 (2H, dd, J=8.1 and 8.0 Hz), 7.08 (1H, d, J=8.6 Hz), 4.51-4.46 (3H, m), 3.56 (2H, t, J=5.7 Hz), 2.73 (2H, t, J=5.7 Hz), 2.12 (2H, d, J=7.1 Hz), 1.94-1.91 (2H, m), 1.76-1.73 (2H, m), 1.67-1.62 (1H, m), 1.39-1.29 (2H, m), 1.12-1.02 (2H, m)
MS (ESI) m/z: 488 (M + H)+.
(Example 6)

trans-6-[3-(2-fluoro-3-methyl-phenyl)-ureido]-3,4-dihydro-1H-isoquinoline-2-carboxylic acid 4-carboxymethyl-cyclohexyl ester

[Formula 6]

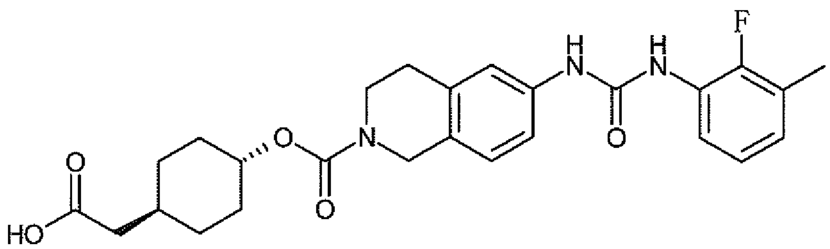

Methyl ester (330 mg, 73%) was obtained in the same way as in Example 3 from the compound (313 mg) obtained in Example (1f) and 2-fluoro-3-methyl-aniline (113 mg) . This methyl ester (330 mg) was hydrolyzed to obtain the title compound (275 mg, 86%) as an off-white solid.
$^1$H NMR (400 MHz, DMSO-d6): δ (ppm)=12.0 (1H, s), 9.02 (1H, s), 8.46 (1H, d, J=2.7 Hz), 7.97 (1H, dd, J=7.4 and 7.4 Hz), 7.32 (1H, s), 7.22 (1H, d, J=9.0 Hz), 7.10 (1H, d, J=8.6 Hz), 7.01 (1H, dd, J=7.8 and 7.8 Hz), 6.88 (1H, dd, J=7.1 and 7.0 Hz), 4.51-4.47 (3H, m), 3.57 (2H, t, J=6.0 Hz), 2.76 (2H, t, J=5.9 Hz), 2.25 (3H, d, J=1.9 Hz), 2.12 (2H, d, J=7.1 Hz), 1.95-1.91 (2H, m), 1.77-1.73 (2H, m), 1.68-1.62 (1H, m), 1.40-1.29 (2H, m), 1.12-1.02 (2H, m)

MS (ESI) m/z: 484 $(M + H)^+$.

(Example 7)

trans-6-[3-(2-fluoro-5-methyl-phenyl)-ureido]-3,4-dihydro-1H-isoquinoline-2-carboxylic acid 4-carboxymethyl-cyclohexyl ester

[Formula 7]

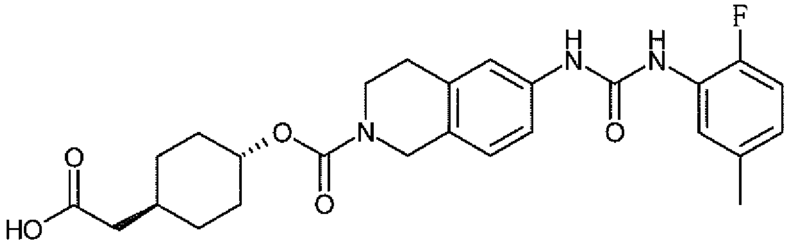

Methyl ester (363 mg, 86%) was obtained in the same way as in Example (1g) from the compound (293 mg) obtained in Example (1f) and 2-fluoro-5-methyl-phenyl isocyanate (0.13 mL). This methyl ester (363 mg) was hydrolyzed to obtain the title compound (338 mg, 96%) as an off-white solid.

$^1H$ NMR (400 MHz, DMSO-d6): δ (ppm)=12.0 (1H, s), 8.99 (1H, s), 8.45 (1H, d, J=2.8 Hz), 8.00 (1H, dd, J=7.8 and 2.0 Hz), 7.34 (1H, s), 7.20 (1H, d, J=8.2 Hz), 7.13-7.08 (2H, m), 6.81-6.78 (1H, m), 4.51-4.47 (3H, m), 3.57 (2H, t, J=5.9 Hz), 2.76 (2H, t, J=5.7 Hz), 2.27 (3H, s), 2.12 (2H, d, J=7.1 Hz), 1.95-1.91 (2H, m), 1.77-1.74 (2H, m), 1.68-1.62 (1H, m), 1.40-1.29 (2H, m), 1.12-1.02 (2H, m)

MS (ESI) m/z: 484 $(M + H)^+$.

(Example 8)

trans-6-[3-(2-fluoro-4-methyl-phenyl)-ureido]-3,4-dihydro-1H-isoquinoline-2-carboxylic acid 4-carboxymethyl-cyclohexyl ester

[Formula 8]

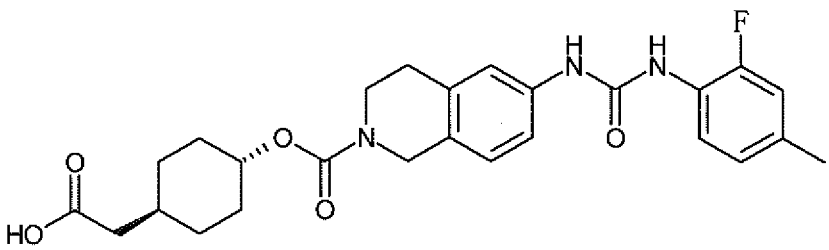

Methyl ester (369 mg, 81%) was obtained in the same way as in Example 3 from the compound (315 mg) obtained in Example (1f) and 2-fluoro-4-methyl-aniline (114 mg). This methyl ester (369 mg) was hydrolyzed to obtain the title compound (307 mg, 86%) as a white solid.

$^1H$ NMR (400 MHz, DMSO-d6): δ (ppm)=12.0 (1H, brs), 8.93 (1H, s), 8.40 (1H, d, J=2.0 Hz), 7.98 (1H dd, J=8.6 and 8.7 Hz), 7.31 (1H, s), 7.21 (1H, d, J=9.4 Hz), 7.08-7.05 (2H, m), 6.94 (1H, d, J=8.6 Hz), 4.51-4.46 (3H, m), 3.57 (2H, t, J=5.9 Hz), 2.75 (2H, t, J=5.5 Hz), 2.26 (3H, s), 2.12 (2H, d, J=7.1 Hz), 1.94-1.91 (2H, m), 1.77-1.73 (2H, m), 1.68-1.62 (1H, m), 1.38-1.30 (2H, m), 1.12-1.02 (2H, m)

MS (ESI) m/z: 484 $(M + H)^+$.

(Example 9)

trans-6-[3-(2-fluoro-6-methyl-phenyl)-ureido]-3,4-dihydro-1H-isoquinoline-2-carboxylic acid 4-carboxymethyl-cyclohexyl ester

[Formula 9]

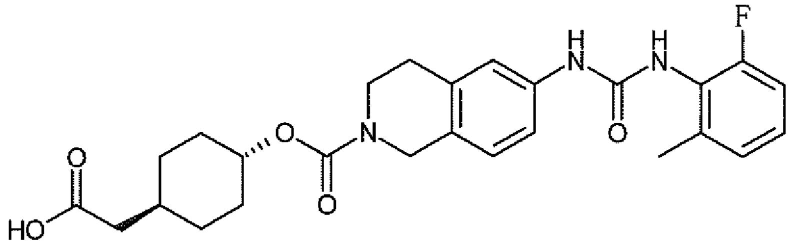

Methyl ester (305 mg, 71%) was obtained in the same way as in Example 3 from the compound (299 mg) obtained in Example (1f) and 2-fluoro-6-methyl-aniline (108 mg). This methyl ester (305 mg) was hydrolyzed to obtain the title compound (269 mg, 91%) as an off-white solid.
H NMR (400 MHz, DMSO-d6): δ (ppm)=12.0 (1H, s), 9.00 (1H, s), 8.07 (1H, s), 7.71 (1H, d, J=8.6 Hz), 7.33 (1H, s), 7.22 (1H, d, J=8.6 Hz), 7.17 (1H, dd, J=15.0 and 8.4 Hz), 7.10 (1H, d, J=8.6 Hz), 6.85 (1H, dd, J=9.0 and 9.0 Hz), 4.51-4.46 (3H, m), 3.57 (2H, t, J=5.8 Hz), 2.76 (2H, t, J=5.9 Hz), 2.14 (3H, d, J=1.5 Hz), 2.12 (2H, d, J=7.1 Hz), 1.95-1.91 (2H, m), 1.77-1.73 (2H, m), 1.68-1.62 (1H, m), 1.39-1.29 (2H, m), 1.12-1.02 (2H, m)
MS (ESI) m/z: 484 $(M + H)^+$.

(Example 10)

trans-6-[3-(3-chloro-2-fluoro-phenyl)-ureido]-3,4-dihydro-1H-isoquinoline-2-carboxylic acid 4-carboxymethyl-cyclohexyl ester

[Formula 10]

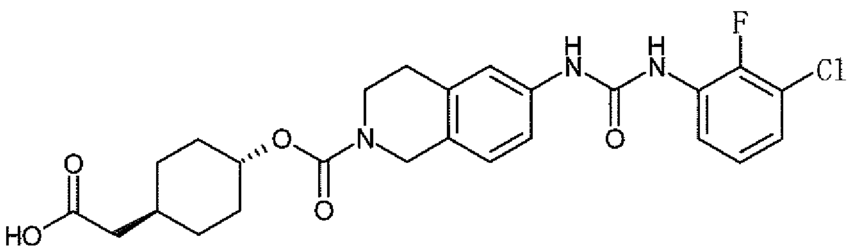

Methyl ester (276 mg, 64%) was obtained in the same way as in Example 3 from the compound (288 mg) obtained in Example (1f) and 3-chloro-2-fluoro-aniline (121 mg). This methyl ester (276 mg) was hydrolyzed to obtain the title compound (243 mg, 91%) as a white solid.
$^1$H NMR (400 MHz, DMSO-d6): 6 (ppm)=12.0 (1H, s), 9.06 (1H, s), 8.69 (1H, d, J=2.8 Hz), 8.14-8.10 (1H, m), 7.32 (1H, s), 7.27-7.14 (3H, m), 7.11 (1H, d, J=8.2 Hz), 4.51-4.47 (3H, m), 3.57 (2H, t, J=6.0 Hz), 2.76 (2H, t, J=5.6 Hz), 2.12 (2H, d, J=7.1 Hz), 1.94-1.91 (2H, m), 1.77-1.74 (2H, m), 1.68-1.61 (1H, m), 1.39-1.29 (2H, m), 1.12-1.02 (2H, m)
MS (ESI) m/z: 504 $(M + H)^+$.
(Example 11)

trans-6-[3-(4-chloro-2-fluoro-phenyl)-ureido]-3,4-dihydro-1H-isoquinoline-2-carboxylic acid 4-carboxymethyl-cyclohexyl ester

[Formula 11]

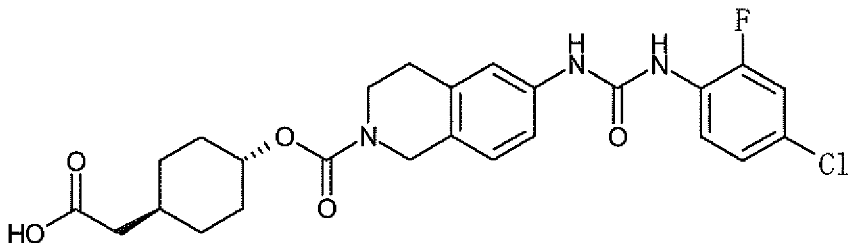

Methyl ester (283 mg, 63%) was obtained in the same way as in Example 3 from the compound (300 mg) obtained in Example (1f) and 4-chloro-2-fluoro-aniline (126 mg). This methyl ester (283 mg) was hydrolyzed to obtain the title compound (257 mg, 94%) as a yellow solid.
$^1$H NMR (400 MHz, DMSO-d6): δ (ppm)=12.0 (1H, brs), 9.04 (1H, s), 8.63 (1H, d, J=2.3 Hz), 8.18 (1H, dd, J=9.0 and 9.0 Hz), 7.47 (1H, dd, J=11.2 and 2.5 Hz), 7.31 (1H, s), 7.25-7.21 (2H, m), 7.11 (1H, d, J=8.3 Hz), 4.49-4.47 (3H, m), 3.57 (2H, t, J=5.9 Hz), 2.76 (2H, t, J=6.1 Hz), 2.12 (2H, d, J=7.1 Hz), 1.95-1.91 (2H, m), 1.77-1.73 (2H, m), 1.68-1.61 (1H, m), 1.39-1.29 (2H, m), 1.12-1.02 (2H, m)
MS (ESI) m/z: 504 $(M + H)^+$.
(Example 12)

trans-6-[3-(5-chloro-2-fluoro-phenyl)-ureido]-3,4-dihydro-1H-isoquinoline-2-carboxylic acid 4-carboxymethyl-cyclohexyl ester

[Formula 12]

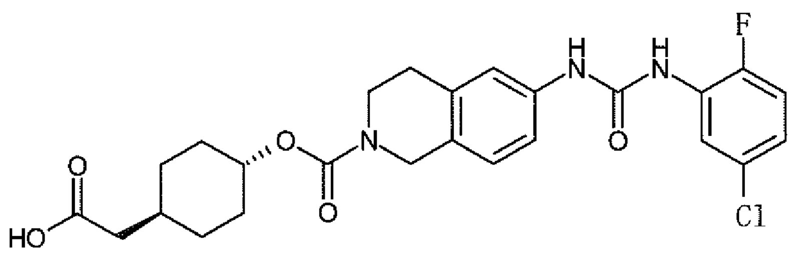

Methyl ester (258 mg, 62%) was obtained in the same way as in Example 3 from the compound (278 mg) obtained in Example (1f) and 5-chloro-2-fluoro-aniline (117 mg). This methyl ester (258 mg) was hydrolyzed to obtain the title compound (201 mg, 80%) as a white solid.
$^1$H NMR (400 MHz, DMSO-d6): δ (ppm)=12.0 (1H, brs), 9.07 (1H, s), 8.73 (1H, d, J=2.8 Hz), 8.29 (1H, dd, J=7.1 and 2.8 Hz), 7.33 (1H, s), 7.31-7.28 (1H, m), 7.21 (1H, d, J=8.2 Hz), 7.12 (1H, d, J=8.2 Hz), 7.07-7.03 (1H, m), 4.49-4.47 (3H, m), 3.57 (2H, t, J=6.0 Hz), 2.77 (2H, t, J=5.9 Hz), 2.12 (2H, d, J=7.1 Hz), 1.95-1.91 (2H, m), 1.78-1.73 (2H, m), 1.68-1.62 (1H, m), 1.40-1.29 (2H, m), 1.12-1.02 (2H, m)
MS (ESI) m/z: 504 $(M + H)^+$.
(Example 13)

trans-6-[3-(2-chloro-6-fluoro-phenyl)-ureido]-3,4-dihydro-1H-isoquinoline-2-carboxylic acid 4-carboxymethyl-cyclohexyl ester

[Formula 13]

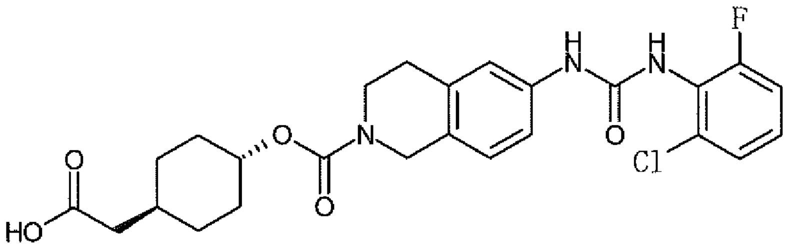

Methyl ester (67 mg, 18%) was obtained in the same way as in Example 3 from the compound (248 mg) obtained in Example (1f) and 2-chloro-6-fluoro-aniline (104 mg). This methyl ester (67 mg) was hydrolyzed to obtain the title compound (50 mg, 76%) as a white solid.
$^1$H NMR (400 MHz, DMSO-d6): δ (ppm)=12.0 (1H, s), 8.90 (1H, s), 8.11 (1H, s), 7.39 (1H, d, J=9.8 Hz), 7.33-7.28 (2H, m), 7.31 (1H, s), 7.23-7.20 (1H, m), 7.08 (1H, d, J=7.8 Hz), 4.49-4.46 (3H, m), 3.56 (2H, t, J=6.2 Hz), 2.74 (2H, t, J=5.9 Hz), 2.12 (2H, d, J=7.0 Hz), 1.94-1.90 (2H, m), 1.77-1.73 (2H, m), 1.67-1.62 (1H, m), 1.39-1.29 (2H, m), 1.12-1.01 (2H, m)
MS (ESI) m/z: 504 $(M + H)^+$.
(Example 14)

trans-6-[3-(2,3,4-trifluoro-phenyl)-ureido]-3,4-dihydro-1H-isoquinoline-2-carboxylic acid 4-carboxymethyl-cyclohexyl ester

[Formula 14]

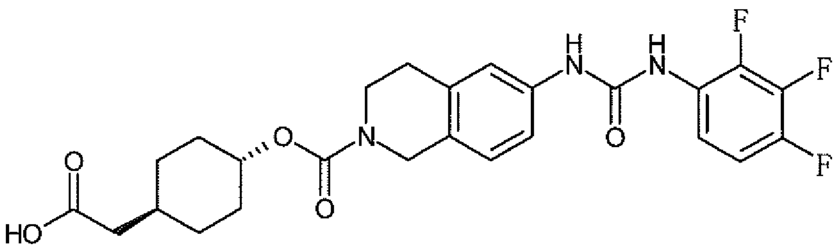

Methyl ester (248 mg, 62%) was obtained in the same way as in Example 3 from the compound (268 mg) obtained in Example (1f) and 2,3,4-trifluoro-aniline (114 mg). This methyl ester (248 mg) was hydrolyzed to obtain the title compound (229 mg, 96%) as a white solid.
$^1$H NMR (400 MHz, DMSO-d6): δ (ppm)=12.0 (1H, brs), 8.99 (1H, s), 8.66 (1H, s), 7.91-7.85 (1H, m), 7.31 (1H, s), 7.28-7.21 (2H, m), 7.11 (1H, d, J=8.2 Hz), 4.49-4.47 (3H, m), 3.57 (2H, t, J=6.2 Hz), 2.76 (2H, t, J=6.2 Hz), 2.12 (2H, d, J=7.1 Hz), 1.94-1.90 (2H, m), 1.77-1.73 (2H, m), 1.68-1.62 (1H, m), 1.38-1.29 (2H, m), 1.12-1.02 (2H, m)
MS (ESI) m/z: 504 $(M - H)^-$.
(Example 15)

trans-6-[3-(2,4,6-trifluoro-phenyl)-ureido]-3,4-dihydro-1H-isoquinoline-2-carboxylic acid 4-carboxymethyl-cyclohexyl ester

[Formula 15]

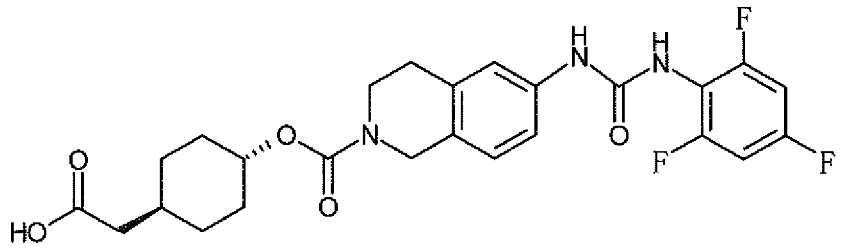

Methyl ester (181 mg, 43%) was obtained in the same way as in Example 3 from the compound (279 mg) obtained in Example (1f) and 2,4,6-trifluoro-aniline (118 mg). This methyl ester (181 mg) was hydrolyzed to obtain the title compound (74 mg, 42%) as a white solid.
$^1$H NMR (400 MHz, DMSO-d6): δ (ppm)=12.1 (1H, s), 8.90 (1H, s), 8.00 (1H, s), 7.29 (1H, s), 7.28-7.21 (3H, m), 7.08 (1H, d, J=8.6 Hz), 4.51-4.46 (3H, m), 3.56 (2H, t, J=5.8 Hz), 2.74 (2H, t, J=5.9 Hz), 2.12 (2H, d, J=7.0 Hz), 1.95-1.91 (2H, m), 1.77-1.74 (2H, m), 1.69-1.62 (1H, m), 1.40-1.29 (2H, m), 1.12-1.02 (2H, m)
MS (ESI) m/z: 504 (M - H)$^-$.
(Example 16)

trans-6-[3-(2-fluoro-4-trifluoromethyl-phenyl)-ureido]-3,4-dihydro-1H-isoquinoline-2-carboxylic acid 4-carboxymethyl-cyclohexyl ester

[Formula 16]

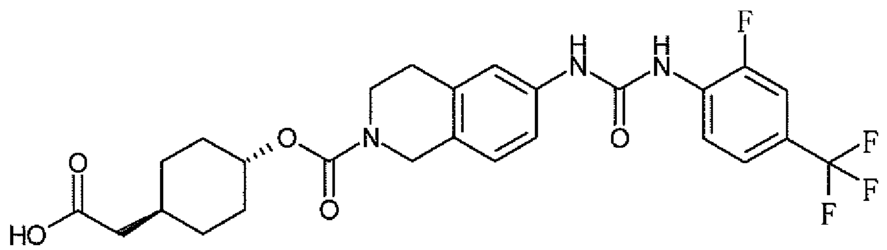

Methyl ester (125 mg, 31%) was obtained in the same way as in Example 3 from the compound (253 mg) obtained in Example (1f) and 2-fluoro-4-trifluoromethyl-aniline (131 mg). This methyl ester (125 mg) was hydrolyzed to obtain the title compound (121 mg, quantitative yield) as an off-white solid.
$^1$H NMR (400 MHz, DMSO-d6): δ (ppm)=12.1 (1H s), 9.15 (1H, s), 8.91 (1H, d, J=2.8 Hz), 8.45 (1H, dd, J=8.2 and 8.2 Hz), 7.71 (1H, d, J=11.7 Hz), 7.54 (1H, d, J=8.6 Hz), 7.33 (1H, s), 7.24 (1H, d, J=8.2 Hz), 7.13 (1H, d, J=8.6 Hz), 4.51-4.48 (3H, m), 3.57 (2H, t, J=5.7 Hz), 2.77 (2H, t, J=5.6 Hz), 2.12 (2H, d, J=7.1 Hz), 1.95-1.91 (2H, m), 1.77-1.74 (2H, m), 1.68-1.62 (1H, m), 1.39-1.30 (2H, m), 1.12-1.02 (2H, m)
MS (ESI) m/z: 536 (M - H)$^-$.
(Example 17)

trans-6-[3-(2-trifluoromethyl-phenyl)-ureido]-3,4-dihydro-1H-isoquinoline-2-carboxylic acid 4-carboxymethyl-cyclohexyl ester

[Formula 17]

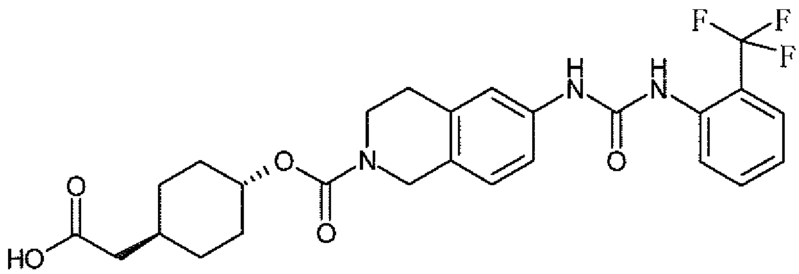

Methyl ester (291 mg, 86%) was obtained in the same way as in Example (1g) from the compound (220 mg) obtained in Example (1f) and 2-trifluoromethyl-phenyl isocyanate (0.12 mL). This methyl ester (291 mg) was hydrolyzed to obtain the title compound (255 mg, 90%) as an off-white solid.
$^1$H NMR (400 MHz, DMSO-d6): δ (ppm)=12.0 (1H, s), 9.32 (1H, s), 8.05 (1H, s), 7.95 (1H, d, J=8.2 Hz), 7.69 (1H, d, J=9.4 Hz), 7.64 (1H, dd, J=7.6 and 7.6 Hz), 7.33 (1H, s), 7.28 (1H, dd, J=8.2 and 8.2 Hz), 7.23 (1H, d, J=9.0 Hz), 7.11 (1H, d, J=8.6 Hz), 4.49-4.47 (3H, m), 3.57 (2H, t, J=5.8 Hz), 2.76 (2H, t, J=5.9 Hz), 2.12 (2H, d, J=7.1 Hz), 1.95-1.91 (2H, m), 1.77-1.73 (2H, m), 1.68-1.62 (1H, m), 1.40-1.29 (2H, m), 1.12-1.02 (2H, m)
MS (ESI) m/z: 518 (M - H)$^-$.

(Example 18)

trans-6-[3-(4-fluoro-2-trifluoromethyl-phenyl)-ureido]-3,4-dihydro-1H-isoquinoline-2-carboxylic acid 4-carboxymethyl-cyclohexyl ester

[Formula 18]

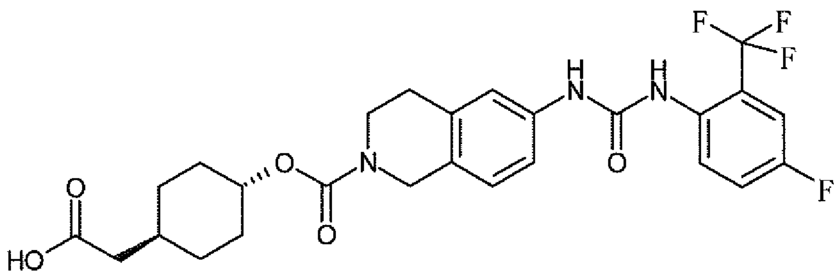

Methyl ester (368 mg, 88%) was obtained in the same way as in Example (1g) from the compound (265 mg) obtained in Example (1f) and 4-fluoro-2-trifluoromethyl-phenyl isocyanate (368 mg). This methyl ester (368 mg) was hydrolyzed to obtain the title compound (330 mg, 92%) as a yellow solid.
$^1$H NMR (400 MHz, DMSO-d5): δ (ppm)=12.1 (1H s), 9.24 (1H, s), 8.07 (1H, s), 7.90 (1H, dd, J=9.0 and 5.0 Hz), 7.60 (1H, dd, J=9.0 and 3.1 Hz), 7.57-7.52 (1H, m), 7.32 (1H s), 7.22 (1H, d, J=8.6 Hz), 7.10 (1H, d, J=8.2 Hz), 4.51-4.46 (3H, m), 3.57 (2H, t, J=5.9 Hz), 2.75 (2H, t, J=5.9 Hz), 2.12 (2H, d, J=7.1 Hz), 1.94-1.91 (2H, m), 1.77-1.73 (2H, m),

1.68-1.62 (1H, m), 1.39-1.29 (2H, m), 1.12-1.02 (2H, m)
MS (ESI) m/z: 536 $(M - H)^-$.

(Example 19)

trans-6-[3-(4-methoxy-2-methyl-phenyl)-ureido]-3,4-dihydro-1H-isoquinoline-2-carboxylic acid 4-carboxymethyl-cyclohexyl ester

[Formula 19]

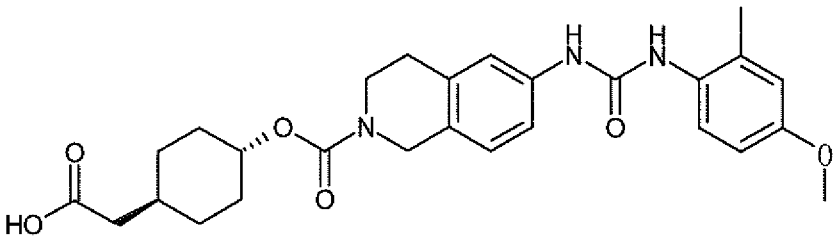

Methyl ester (337 mg, 81%) was obtained in the same way as in Example (1g) from the compound (284 mg) obtained in Example (1f) and 4-methoxy-2-methylphenyl-isocyanate (337 mg). This methyl ester (337 mg) was hydrolyzed to obtain the title compound (314 mg, 96%) as a white solid.
$^1$H NMR (400 MHz, DMSO-d6): δ (ppm)=12.1 (1H, s), 8.78 (1H, s), 7.76 (1H, s), 7.54 (1H, d, J=9.0 Hz), 7.31 (1H, s), 7.21 (1H, d, J=6.6 Hz), 7.07 (1H, d, J=8.6 Hz), 6.79 (1H, d, J=3.2 Hz), 6.73 (1H, dd, J=8.6 and 3.1 Hz), 4.51-4.45 (3H, m), 3.72 (3H, s), 3.56 (2H, t, J=5.9 Hz), 2.74 (2H, t, J=5.6 Hz), 2.20 (3H, s), 2.12 (2H, d, J=7.1 Hz), 1.95-1.91 (2H, m), 1.77-1.73 (2H, m), 1.68-1.62 (1H, m), 1.39-1.29 (2H, m), 1.12-1.02 (2H, m)
MS (ESI) m/z: 496 $(M + H)^+$.

(Example 20)

trans-6-[3-(2-chloro-4-methyl-phenyl)-ureido]-3,4-dihydro-1H-isoquinoline-2-carboxylic acid 4-carboxymethyl-cyclohexyl ester

[Formula 20]

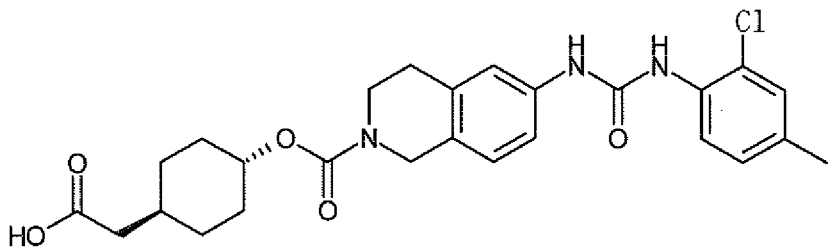

Methyl ester (343 mg, 94%) was obtained in the same way as in Example (1g) from the compound (246 mg) obtained in Example (1f) and 2-chloro-4-methyl-phenyl isocyanate (143 mg). This methyl ester (343 mg) was hydrolyzed to obtain the title compound (296 mg, 89%) as a white solid.

$^1$H NMR (400 MHz, DMSO-d6): δ (ppm)=12.1 (1H, s), 9.27 (1H, s), 8.19 (1H, s), 8.01 (1H, d, J=8.6 Hz), 7.33 (1H, s), 7.29 (1H, s), 7.22 (1H, d, J=8.2 Hz), 7.10 (2H, d, J=8.6 Hz), 4.51-4.47 (3H, m), 3.57 (2H, t, J=6.0 Hz), 2.76 (2H, t, J=5.9 Hz), 2.26 (3H, s), 2.12 (2H, d, J=7.1 Hz), 1.95-1.91 (2H, m), 1.78-1.73 (2H, m), 1.68-1.62 (1H, m), 1.39-1.29 (2H, m), 1.39-1.29 (2H, m)
MS (ESI) m/z: 500 $(M + H)^+$.

(Example 21)

trans-6-[3-(2-fluoro-3-trifluoromethyl-phenyl)-ureido]-3,4-dihydro-1H-isoquinoline-2-carboxylic acid 4-carboxymethyl-cyclohexyl ester

[Formula 21]

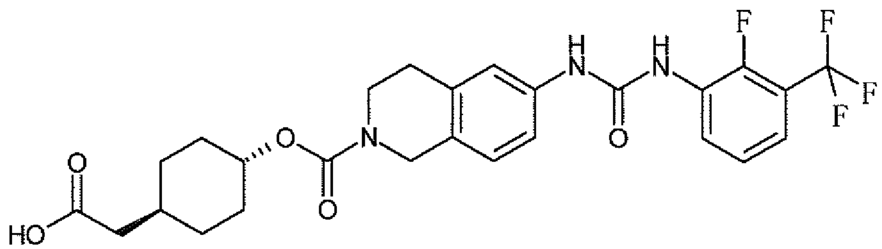

Methyl ester (448 mg, 87%) was obtained in the same way as in Example (1g) from the compound (322 mg) obtained in Example (1f) and 2-fluoro-3-trifluoromethyl-phenyl isocyanate (0.16 mL). This methyl ester (448 mg) was hydrolyzed to obtain the title compound (397 mg, 91%) as a pale yellow solid.
$^1$H NMR (400 MHz, DMSO-d6): δ (ppm)=12.0 (1H, s), 9.09 (1H, s), 8.82 (1H, d, J=2.7 Hz), 8.48-8.43 (1H, m), 7.38-7.33 (2H, m), 7.35 (1H, s), 7.24 (1H, d, J=8.2 Hz), 7.12 (1H, d, J=8.2 Hz), 4.52-4.46 (3H, m), 3.57 (2H, t, J=5.9 Hz), 2.77 (2H, t, J=5.9 Hz), 2.12 (2H, d, J=6.7 Hz), 1.95-1.91 (2H, m), 1.77-1.73 (2H, m), 1.67-1.62 (1H, m), 1.39-1.29 (2H, m), 1.12-1.02 (2H, m)
MS (ESI) m/z: 536 $(M - H)^-$.
(Example 22)

trans-6-[3-(2-fluoro-5-trifluoromethyl-phenyl)-ureido]-3,4-dihydro-1H-isoquinoline-2-carboxylic acid 4-carboxymethyl-cyclohexyl ester

[Formula 22]

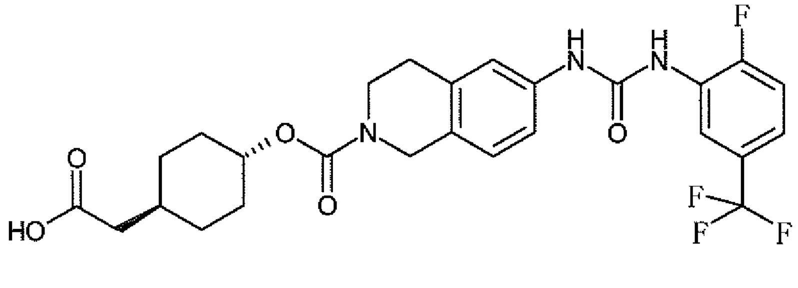

Methyl ester (426 mg, 88%) was obtained in the same way as in Example (1g) from the compound (305 mg) obtained in Example (1f) and 2-fluoro-5-trifluoromethyl-phenyl isocyanate (0.15 mL). This methyl ester (426 mg) was hydrolyzed to obtain the title compound (381 mg, 92%) as a pale yellow solid.
$^1$H NMR (400 MHz, DMSO-d6): δ (ppm)=12.1 (1H, s), 9.13 (1H, s), 8.88 (1H, d, J=2.7 Hz), 8.63 (1H, dd, J=7.6 and 1.8 Hz), 7.50 (1H, dd, J=9.8 and 9.8 Hz), 7.41-7.36 (1H, m), 7.36 (1H, s), 7.21 (1H, d, J=8.3 Hz), 7.12 (1H, d, J=8.2 Hz), 4.51-4.47 (3H, m), 3.57 (2H, t, J=5.9 Hz), 2.77 (2H, t, J=5.9 Hz), 2.12 (2H, d, J=6.7 Hz), 1.95-1.91 (2H, m), 1.77-1.74 (2H, m), 1.68-1.62 (1H, m), 1.40-1.30 (2H, m), 1.12-1.02 (2H, m)
MS (ESI) m/z: 538 $(M + H)^+$.

(Example 23)

trans-6-[3-(2,4-dimethyl-phenyl)-ureido]-3,4-dihydro-1H-isoquinoline-2-carboxylic acid 4-carboxymethyl-cyclohexyl ester

[Formula 23]

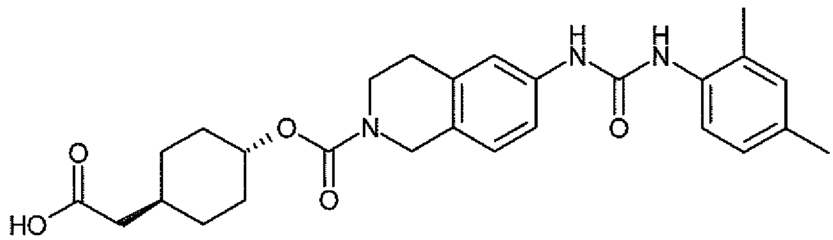

Methyl ester (412 mg, 84%) was obtained in the same way as in Example (1g) from the compound (342 mg) obtained in Example (1f) and 2,4-dimethyl-phenyl isocyanate (0.25 mL). This methyl ester (412 mg) was hydrolyzed to obtain the title compound (384 mg, 96%) as a white solid.
$^1$H NMR (400 MHz, DMSO-d6) : δ (ppm)=12.0 (1H, s), 8.86 (1H, s), 7.81 (1H, s), 7.66 (1H, d, J=8.2 Hz), 7.32 (1H, s), 7.21 (1H, d, J=8.2 Hz), 7.08 (1H, d, J=8.2 Hz), 6.99 (1H, s), 6.94 (1H, d, J=8.2 Hz), 4.48-4.46 (3H, m), 3.56 (2H, t, J=6.1 Hz), 2.75 (2H, t, J=5.9 Hz), 2.22 (3H, s), 2.22 (3H, s), 2.12 (2H, d, J=7.1 Hz), 1.95-1.91 (2H, m), 1.77-1.73 (2H, m), 1.68-1.62 (1H, m), 1.39-1.29 (2H, m), 1.12-1.02 (2H, m)
MS (ESI) m/z: 480 $(M + H)^+$.

(Example 24)

trans-6-[3-(2,4-dichloro-phenyl)-ureido]-3,4-dihydro-1H-isoquinoline-2-carboxylic acid 4-carboxymethyl-cyclohexyl ester

[Formula 24]

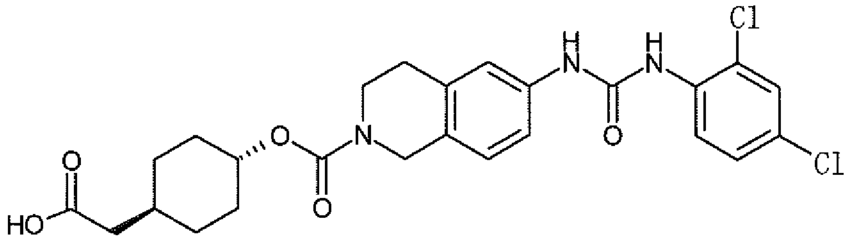

Methyl ester (393 mg, 88%) was obtained in the same way as in Example (1g) from the compound (290 mg) obtained in Example (1f) and 2,4-dichloro-phenyl isocyanate (189 mg). This methyl ester (393 mg) was hydrolyzed to obtain the title compound (358 mg, 94%) as a pale yellow solid.
$^1$H NMR (400 MHz, DMSO-d6): δ (ppm)=12.0 (1H, s), 9.38 (1H, s), 8.37 (1H, s), 8.21 (1H, d, J=9.0 Hz), 7.63 (1H, d, J=2.7 Hz), 7.38 (1H, dd, J=9.0 and 2.4 Hz), 7.32 (1H, s), 7.23 (1H, d, J=9.0 Hz), 7.11 (1H, d, J=8.6 Hz), 4.51-4.47 (3H, m), 3.57 (2H, t, J=6.1 Hz), 2.76 (2H, t, J=5.9 Hz), 2.12 (2H, d, J=7.1 Hz), 1.95-1.91 (2H, m), 1.77-1.73 (2H, m), 1.68-1.62 (1H m), 1.39-1.29 (2H, m), 1.13-1.02 (2H, m)
MS (ESI) m/z: 521 $(M + H)^+$.

(Example 25)

trans-6-[3-(2-fluoro-6-trifluoromethyl-phenyl)-ureido]-3,4-dihydro-1H-isoquinoline-2-carboxylic acid 4-carboxymethyl-cyclohexyl ester

[Formula 25]

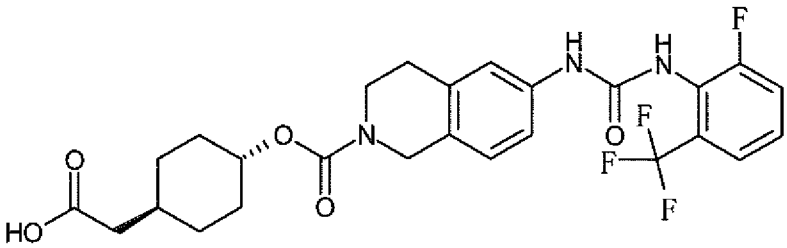

Methyl ester (301 mg, 87%) was obtained in the same way as in Example (1g) from the compound (218 mg) obtained in Example (1f) and 2-fluoro-6-trifluoromethyl-phenyl isocyanate (0.11 mL). This methyl ester (301 mg) was hydrolyzed to obtain the title compound (265 mg, 90%) as a pale yellow solid.
$^1$H NMR (400 MHz, DMSO-d6): δ (ppm)=12.1 (1H, s), 8.96 (1H, s), 8.03 (1H, s), 7.67-7.60 (2H, m), 7.56-7.51 (1H, m), 7.32 (1H, s), 7.21 (1H, dd, J=7.8 and 1.1 Hz), 7.09 (1H, d, J=8.6 Hz), 4.51-4.46 (3H, m), 3.56 (2H, t, J=5.9 Hz), 2.74 (2H, t, J=5.9 Hz), 2.12 (2H, d, J=7.1 Hz), 1.95-1.91 (2H, m), 1.77-1.73 (2H, m), 1.68-1.62 (1H, m), 1.39-1.29 (2H, m), 1.12-1.01 (2H, m)
MS (ESI) m/z: 538 $(M + H)^+$.
(Example 26)

trans-6-[3-(4-chloro-2-methyl-phenyl)-ureido]-3,4-dihydro-1H-isoquinoline-2-carboxylic acid 4-carboxymethyl-cyclohexyl ester

[Formula 26]

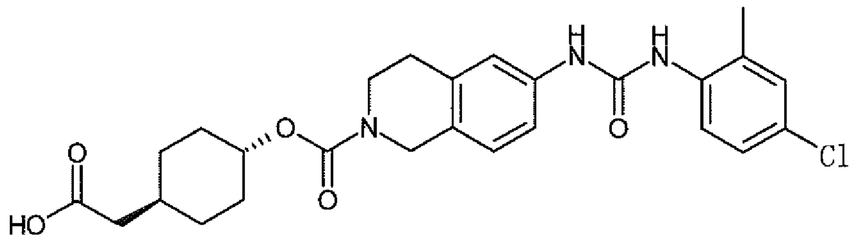

Methyl ester (218 mg, 89%) was obtained in the same way as in Example (1g) from the compound (166 mg) obtained in Example (1f) and 4-chloro-2-methyl-phenyl isocyanate (96 mg). This methyl ester (218 mg) was hydrolyzed to obtain the title compound (190 mg, 90%) as a pale yellow solid.
$^{1}$H NMR (400 MHz, DMSO-d6): δ (ppm)=12.1 (1H, s), 9.01 (1H, s), 7.97 (1H, s), 7.90 (1H, d, J=8.6 Hz), 7.33 (1H, s), 7.27 (1H, d, J=2.3 Hz), 7.23-7.18 (2H, m), 7.10 (1H, d, J=8.2 Hz), 4.49-4.46 (3H, m), 3.57 (2H, t, J=5.8 Hz), 2.76 (2H, t, J=5.5 Hz), 2.24 (3H, s), 2.12 (2H, d, J=7.1 Hz), 1.95-1.91 (2H, m), 1.77-1.73 (2H, m), 1.68-1.62 (1H, m), 1.40-1.29 (2H, m), 1.12-1.02 (2H, m)
MS (ESI) m/z: 500 $(M + H)^+$.
(Example 27)

trans-6-[3-(2-chloro-3-methyl-phenyl)-ureido]-3,4-dihydro-1H-isoquinoline-2-carboxylic acid 4-carboxymethyl-cyclohexyl ester

[Formula 27]

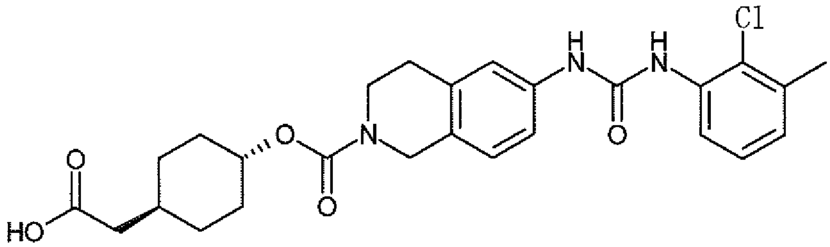

A tetrahydrofuran (4 mL) solution of 2-chloro-3-methylbenzoic acid (127 mg), DPPA (0.2 mL), and triethylamine (0.15 mL) was heated to reflux for 3 hours. A tetrahydrofuran (4 mL) solution of the compound (257 mg) obtained in Example (1f) was added to the reaction mixture. The reaction mixture was further heated to reflux for 1 hour, then cooled to room temperature, and then concentrated. The residue was purified by chromatography (dichloromethane:ethyl acetate=1:0→4:1) to obtain methyl ester (263 mg, 69%). To a tetrahydrofuran (2 mL)/methanol (2 mL) mixed solution of this methyl ester (263 mg), an aqueous sodium hydroxide solution (1.0 mol/L aqueous solution, 2.2 mL) was added at room temperature. The reaction mixture was stirred for 16 hours and concentrated. The residue was diluted with water, then neutralized with a 1 N aqueous hydrochloric acid solution (2.5 mL), and collected by filtration to obtain the title compound (245 mg, 96%) as an off-white solid.
$^{1}$H NMR (400 MHz, DMSO-d6): δ (ppm)=12.1 (1H, s), 9.37 (1H, s), 8.26 (1H, s), 8.01 (1H, d, J=8.3 Hz), 7.34 (1H, s), 7.24-7.17 (2H, m), 7.11 (1H, d, J=8.6 Hz), 7.01 (1H, d, J=7.5 Hz), 4.48-4.47 (3H, m), 3.57 (2H, t, J=6.0 Hz), 2.76 (2H, t, J=5.9 Hz), 2.35 (3H, s), 2.12 (2H, d, J=7.1 Hz), 1.95-1.91 (2H, m), 1.77-1.73 (2H, m), 1.68-1.62 (1H, m), 1.40-1.30

(2H, m), 1.12-1.02 (2H, m)
MS (ESI) m/z: 500 (M + H)+.

(Example 28)

trans-6-[3-(4-chloro-2-trifluoromethyl-phenyl)-ureido]-3,4-dihydro-1H-isoquinoline-2-carboxylic acid 4-carboxymethyl-cyclohexyl ester

[Formula 28]

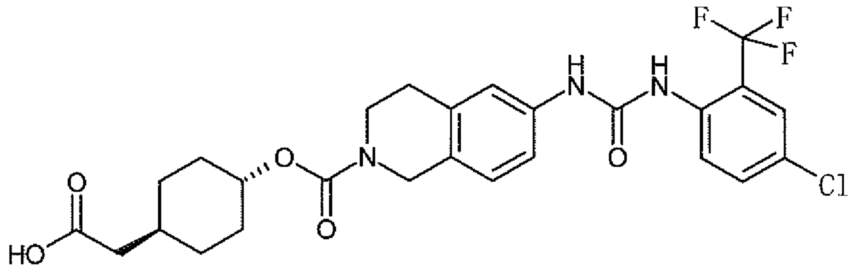

Methyl ester (326 mg, 92%) was obtained in the same way as in Example (1g) from the compound (217 mg) obtained in Example (1f) and 4-chloro-2-trifluoromethyl-phenyl isocyanate (0.11 mL). This methyl ester (326 mg) was hydrolyzed to obtain the title compound (285 mg, 90%) as a yellow solid.
$^1$H NMR (400 MHz, DMSO-d6): δ (ppm)=12.0 (1H, s), 9.38 (1H, s), 8.14 (1H, s), 8.01 (1H, d, J=9.0 Hz), 7.74 (1H, dd, J=2.6 and 2.5 Hz), 7.72 (1H, dd, J=8.8 and 2.5 Hz), 7.32 (1H, s), 7.23 (1H, d, J=7.1 Hz), 7.11 (1H, d, J=8.6 Hz), 4.51-4.47 (3H, m), 3.57 (2H, t, J=5.9 Hz), 2.76 (2H, t, J=5.9 Hz), 2.12 (2H, d, J=7.1 Hz), 1.95-1.91 (2H, m), 1.76-1.74 (2H, m), 1.68-1.62 (1H, m), 1.39-1.29 (2H, m), 1.12-1.02 (2H, m)
MS (ESI) m/z: 554 (M + H)+.

(Example 29)

trans-6-[3-(2-chloro-4-trifluoromethyl-phenyl)-ureido]-3,4-dihydro-1H-isoquinoline-2-carboxylic acid 4-carboxymethyl-cyclohexyl ester

[Formula 29]

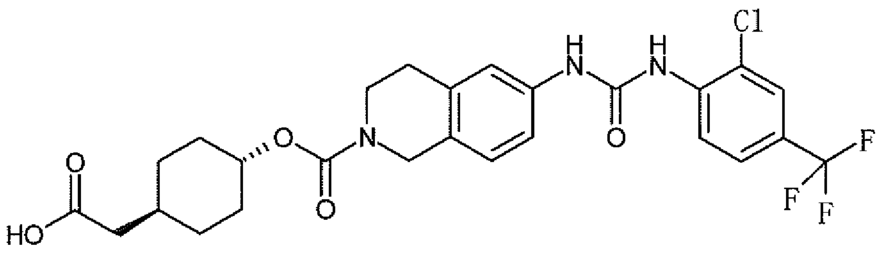

Methyl ester (344 mg, 84%) was obtained in the same way as in Example (1g) from the compound (249 mg) obtained in Example (1f) and 2-chloro-4-trifluoromethyl-phenyl isocyanate (0.13 mL). This methyl ester (344 mg) was hydrolyzed to obtain the title compound (298 mg, 89%) as a white solid.
$^1$H NMR (400 MHz, DMSO-d6): δ (ppm)=12.0 (1H, s), 9.56 (1H, s), 8.61 (1H, s), 8.48 (1H, d, J=9.0 Hz), 7.88 (1H, d, J=1.6 Hz), 7.69 (1H, dd, J=9.0 and 1.5 Hz), 7.35 (1H, s), 7.25 (1H, d, J=7.9 Hz), 7.14 (1H, d, J=8.2 Hz), 4.51-4.48 (3H, m), 3.58 (2H, t, J=5.9 Hz), 2.78 (2H, t, J=5.7 Hz), 2.12 (2H, d, J=7.0 Hz), 1.95-1.91 (2H, m), 1.77-1.74 (2H, m), 1.68-1.63

(1H, m), 1.40-1.30 (2H, m), 1.12-1.02 (2H, m)
MS (ESI) m/z: 554 $(M + H)^+$.

(Example 30)

trans-6-[3-(3-fluoro-4-methyl-phenyl)-ureido]-3,4-dihydro-1H-isoquinoline-2-carboxylic acid 4-carboxymethyl-cyclohexyl ester

[Formula 30]

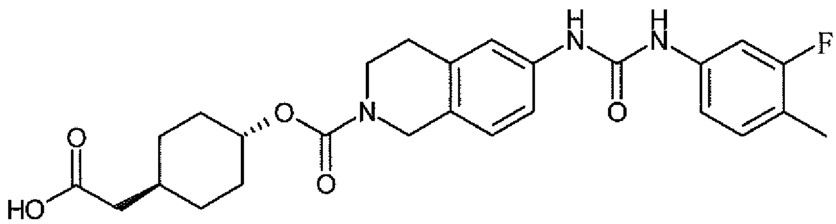

Methyl ester (373 mg, 99%) was obtained in the same way as in Example (1g) from the compound (262 mg) obtained in Example (1f) and 3-fluoro-4-methyl-phenyl isocyanate (0.12 mL). This methyl ester (373 mg) was hydrolyzed to obtain the title compound (278 mg, 77%) as a pale yellow solid.
$^1H$ NMR (400 MHz, DMSO-d6): δ (ppm)=12.0 (1H, brs), 8.75 (1H, s), 8.62 (1H, s), 7.43 (1H, dd, J=12.7 and 2.1 Hz), 7.31 (1H, s), 7.20 (1H, dd, J=8.0 and 8.0 Hz), 7.15 (1H, d, J=8.6 Hz), 7.09 (1H, d, J=8.6 Hz), 7.01 (1H, dd, J=8.4 and 2.1 Hz), 4.49-4.46 (3H, m), 3.56 (2H, t, J=6.1 Hz), 2.75 (2H, t, J=5.9 Hz), 2.16 (3H, s), 2.12 (2H, d, J=7.1 Hz), 1.95-1.91 (2H, m), 1.77-1.73 (2H, m), 1.68-1.62 (1H, m), 1.39-1.29 (2H, m), 1.12-1.02 (2H, m)
MS (ESI) m/z: 482 $(M - H)^-$.

(Example 31)

trans-6-[3-(3,5-difluoro-phenyl)-ureido]-3,4-dihydro-1H-isoquinoline-2-carboxylic acid 4-carboxymethyl-cyclohexyl ester

[Formula 31]

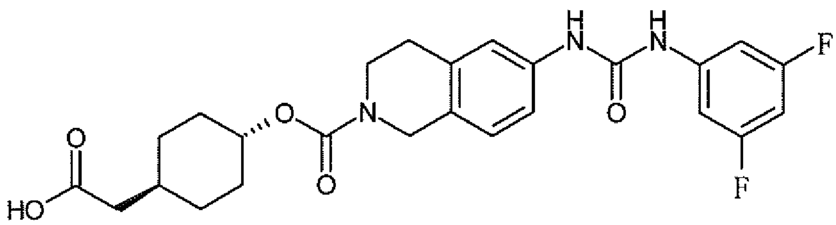

Methyl ester (107 mg, 84%) was obtained in the same way as in Example (1g) from the compound (235 mg) obtained in Example (1f) and 3,5-difluoro-phenyl isocyanate (0.10 mL). This methyl ester (107 mg) was hydrolyzed to obtain the title compound (89 mg, 86%) as a pale yellow solid.
$^1H$ NMR (400 MHz, DMSO-d6): δ (ppm)=12.1 (1H brs), 9.07 (1H, s), 8.79 (1H, s), 7.32 (1H, s), 7.24-7.18 (3H, m), 7.11 (1H, d, J=8.6 Hz), 6.79 (1H, dd, J=9.5 and 9.5 Hz), 4.50-4.47 (3H, m), 3.57 (2H, t, J=5.6 Hz), 2.76 (2H, t, J=6.0 Hz), 2.12 (2H, d, J=7.0 Hz), 1.95-1.91 (2H, m), 1.77-1.73 (2H, m), 1.68-1.63 (1H, m), 1.39-1.29 (2H, m), 1.12-1.01 (2H, m)
MS (ESI) m/z: 486 (M - H) .

(Example 32)

trans-6-[3-(3-chloro-4-fluoro-phenyl)-ureido]-3,4-dihydro-1H-isoquinoline-2-carboxylic acid 4-carboxymethyl-cyclohexyl ester

[Formula 32]

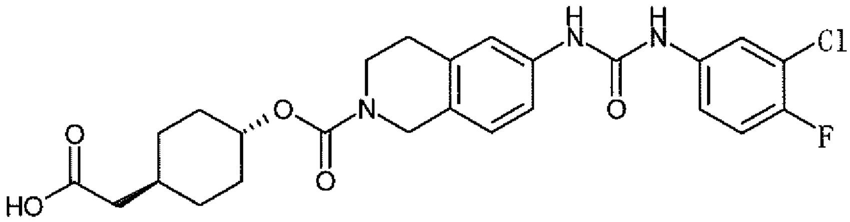

Methyl ester (252 mg, 52%) was obtained in the same way as in Example (1g) from the compound (273 mg) obtained in Example (1f) and 3-chloro-4-fluoro-phenyl isocyanate (0.12 mL). This methyl ester (252 mg) was hydrolyzed to obtain the title compound (227 mg, 93%) as a pale yellow solid.
$^1$H NMR (400 MHz, DMSO-d6): δ ppm)=12.0 (1H, s), 8.85 (1H, s), 8.69 (1H, s), 7.81 (1H, dd, J=6.8 and 2.5 Hz), 7.36-7.27 (3H, m), 7.22 (1H, d, J=7.5 Hz), 7.09 (1H, d, J=8.2 Hz), 4.51-4.46 (3H, m), 3.57 (2H, t, J=6.0 Hz), 2.75 (2H, t, J=5.9 Hz), 2.12 (2H, d, J=6.7 Hz), 1.95-1.91 (2H, m), 1.77-1.73 (2H, m), 1.68-1.62 (1H, m), 1.39-1.29 (2H, m), 1.12-1.02 (2H, m)
MS (ESI) m/z: 504 $(M + H)^+$.
(Example 33)

trans-6-[3-(3,5-dimethyl-phenyl)-ureido]-3,4-dihydro-1H-isoquinoline-2-carboxylic acid 4-carboxymethyl-cyclohexyl ester

[Formula 33]

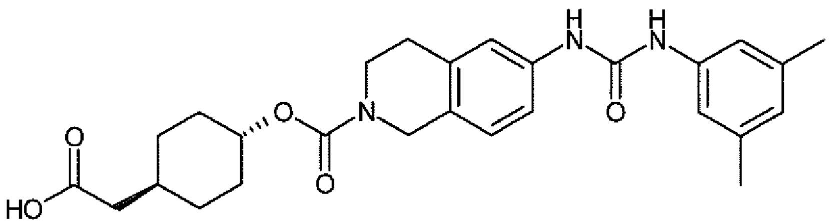

Methyl ester (348 mg, 92%) was obtained in the same way as in Example (1g) from the compound (265 mg) obtained in Example (1f) and 3,5-dimethyl-phenyl isocyanate (0.13 mL). This methyl ester (348 mg) was hydrolyzed to obtain the title compound (313 mg, 93%) as a yellow solid.
$^1$H NMR (400 MHz, DMSO-d6): δ (ppm)=12.0 (1H, s), 8.56 (1H, s), 8.48 (1H, s), 7.33 (1H, s), 7.20 (1H, d, J=7.0 Hz), 7.08 (1H, d, J=8.6 Hz), 7.07 (2H, s), 6.61 (1H, s), 4.48-4.46 (3H, m), 3.57 (2H, t, J=5.5 Hz), 2.75 (2H, t, J=5.6 Hz), 2.23 (6H, s), 2.12 (2H, d, J=7.1 Hz), 1.95-1.91 (2H, m), 1.78-1.73 (2H, m), 1.69-1.62 (1H, m), 1.39-1.29 (2H, m), 1.12-1.02 (2H, m)
MS (ESI) m/z: 478 $(M - H)^-$.
(Example 34)

trans-6-[3-(3-chloro-4-methyl-phenyl)-ureido]-3,4-dihydro-1H-isoquinoline-2-carboxylic acid 4-carboxymethyl-cyclohexyl ester

[Formula 34]

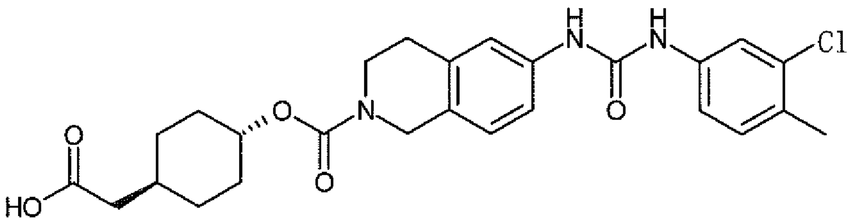

Methyl ester (317 mg, 94%) was obtained in the same way as in Example (1g) from the compound (228 mg) obtained in Example (1f) and 3-chloro-4-methyl-phenyl isocyanate (0.11 mL). This methyl ester (317 mg) was hydrolyzed to obtain the title compound (282 mg, 92%) as a yellow solid.
$^1$H NMR (400 MHz, DMSO-d6): δ (ppm)=12.1 (1H, s), 8.75 (1H, s), 8.64 (1H, s), 7.70 (1H, d, J=2.0 Hz), 7.32 (1H, s), 7.25-7.20 (2H, m), 7.17 (1H, dd, J=8.2 and 1.9 Hz), 7.09 (1H, d, J=8.2 Hz), 4.48-4.46 (3H, m), 3.57 (2H, t, J=5.6 Hz), 2.75 (2H, t, J=5.5 Hz), 2.26 (3H, s), 2.12 (2H, d, J=7.1 Hz), 1.94-1.91 (2H, m), 1.77-1.73 (2H, m), 1.68-1.62 (1H, m), 1.39-1.29 (2H, m), 1.12-1.02 (2H, m)
MS (ESI) m/z: 500 $(M + H)^+$.
(Example 35)

trans-6-[3-(2,3-dimethyl-phenyl)-ureido]-3,4-dihydro-1H-isoquinoline-2-carboxylic acid 4-carboxymethyl-cyclohexyl ester

[Formula 35]

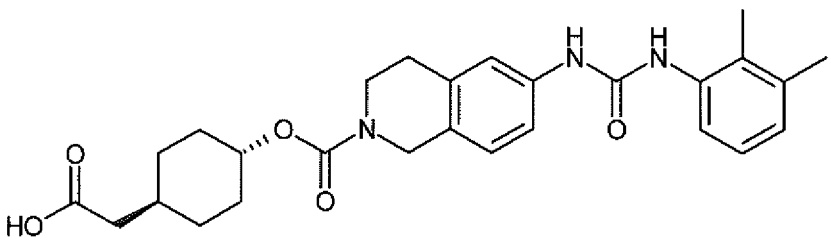

Methyl ester (312 mg, 92%) was obtained in the same way as in Example (1g) from the compound (237 mg) obtained in Example (1f) and 2,3-dimethyl-phenyl isocyanate (0.12 mL). This methyl ester (312 mg) was hydrolyzed to obtain the title compound (258 mg, 85%) as a white solid.
$^1$H NMR (400 MHz, DMSO-d6): δ (ppm)=12.1 (1H, s), 8.87 (1H, s), 7.92 (1H, s), 7.54 (1H, d, J=8.3 Hz), 7.33 (1H, s), 7.22 (1H, d, J=7.9 Hz), 7.08 (1H, d, J=8.6 Hz), 7.03 (1H, dd, J=7.6 and 7.6 Hz), 6.90 (1H, d, J=7.4 Hz), 4.51-4.46 (3H, m), 3.57 (2H, t, J=6.1 Hz), 2.75 (2H, t, J=5.9 Hz), 2.25 (3H, s), 2.13 (3H, s), 2.12 (2H, d, J=5.9 Hz), 1.95-1.91 (2H, m), 1.77-1.74 (2H, m), 1.68-1.63 (1H, m), 1.40-1.30 (2H, m), 1.12-1.02 (2H, m)
MS (ESI) m/z: 478 $(M - H)^-$.
(Example 36)

trans-6-[3-(3-chloro-2-methyl-phenyl)-ureido]-3,-4-dihydro-1H-isoquinoline-2-carboxylic acid 4-carboxymethyl-cyclohexyl ester

[Formula 36]

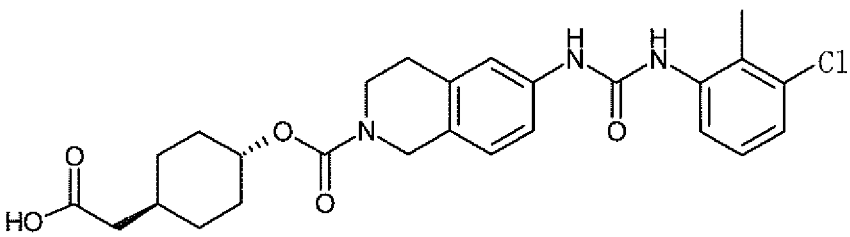

Methyl ester (366 mg, 91%) was obtained in the same way as in Example (1g) from the compound (271 mg) obtained in Example (1f) and 3-chloro-2-methylphenyl isocyanate (0.13 mL). This methyl ester (366 mg) was hydrolyzed to obtain the title compound (329 mg, 92%) as a white solid.
$^1$H NMR (400 MHz, DMSO-d6): δ (ppm)=12.0 (1H, s), 8.98 (1H, s), 8.11 (1H, s), 7.76 (1H, dd, J=7.2 and 2.2 Hz), 7.32 (1H, s), 7.22 (1H, d, J=9.4 Hz), 7.19-7.13 (2H, m), 7.09 (1H, d, J=8.2 Hz), 4.52-4.46 (3H, m), 3.57 (2H, t, J=6.1 Hz), 2.75 (2H, t, J=6.1 Hz), 2.29 (3H, s), 2.12 (2H, d, J=7.1 Hz), 1.95-1.91 (2H, m), 1.77-1.73 (2H, m), 1.68-1.62 (1H, m), 1.39-1.29 (2H, m), 1.12-1.02 (2H, m)
MS (ESI) m/z: 498 (M - H) .
(Example 37)

trans-6-[3-(2,3-dichloro-phenyl)-ureido]-3,4-dihydro-1H-isoquinoline-2-carboxylic acid 4-carboxymethyl-cyclohexyl ester

[Formula 37]

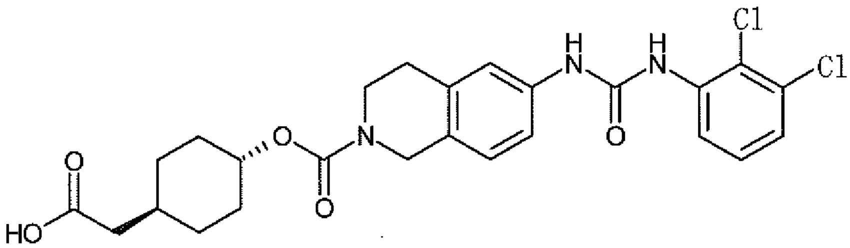

Methyl ester (386 mg, 93%) was obtained in the same way as in Example (1g) from the compound (268 mg) obtained in Example (1f) and 2,3-dichloro-phenyl isocyanate (0.12 mL). This methyl ester (386 mg) was hydrolyzed to obtain the title compound (338 mg, 90%) as a white solid.
$^1$H NMR (400 MHz, DMSO-d6): δ (ppm)=12.1 (1H, s), 9.48 (1H, s), 8.47 (1H, s), 8.17 (1H, dd, J=8.0 and 1.8 Hz), 7.35-7.27 (2H, m), 7.34 (1H s), 7.24 (1H, d, J=8.6 Hz), 7.12 (1H, d, J=8.6 Hz), 4.51-4.47 (3H, m), 3.57 (2H, t, J=5.9 Hz), 2.77 (2H, t, J=5.7 Hz), 2.12 (2H, d, J=7.1 Hz), 1.95-1.91 (2H, m), 1.77-1.74 (2H, m), 1.68-1.63 (1H, m), 1.40-1.29 (2H, m), 1.12-1.02 (2H, m)
MS (ESI) m/z: 519 (M - H)$^-$.
(Example 38)

trans-6-[3-(4-fluoro-3-methyl-phenyl)-ureido]-3,4-dihydro-1H-isoquinoline-2-carboxylic acid 4-carboxymethyl-cyclohexyl ester

[Formula 38]

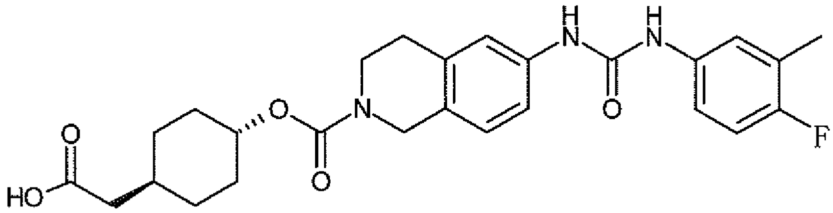

Methyl ester (395 mg, 94%) was obtained in the same way as in Example (1g) from the compound (293 mg) obtained in Example (1f) and 4-fluoro-3-methyl-phenyl isocyanate (0.13 mL). This methyl ester (395 mg) was hydrolyzed to obtain the title compound (356 mg, 93%) as a pale yellow solid.
$^1$H NMR (400 MHz, DMSO-d6): δ (ppm)=12.0 (1H, brs), 8.93 (1H, s), 8.92 (1H, s), 7.36 (1H, dd, J=7.4 and 2.3 Hz), 7.30 (1H, d, J=2.0 Hz), 7.25-7.19 (2H, m), 7.05 (1H, d, J=8.6 Hz), 7.01 (1H, dd, J=9.2 and 9.2 Hz), 4.50-4.44 (3H, m), 3.54 (2H, t, J=6.0 Hz), 2.72 (2H, t, J=5.9 Hz), 2.18 (3H, d, J=2.0 Hz), 2.08 (2H, d, J=6.7 Hz), 1.92-1.89 (2H, m), 1.75-1.72 (2H, m), 1.66-1.61 (1H, m), 1.37-1.27 (2H, m), 1.09-0.99 (2H, m)
MS (ESI) m/z: 482 (M - H) .
(Example 39)

trans-6-[3-(3,5-dimethoxy-phenyl)-ureido]-3,4-dihydro-1H-isoquinoline-2-carboxylic acid 4-carboxymethyl-cyclohexyl ester

[Formula 39]

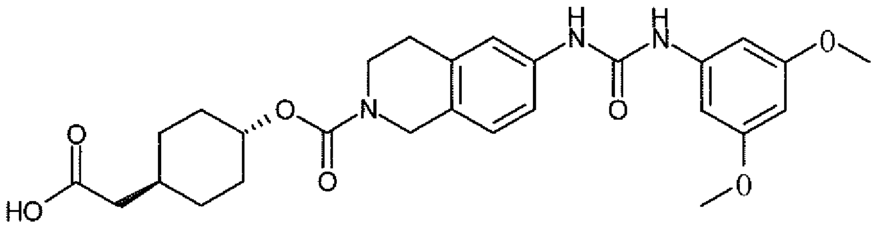

Methyl ester (203 mg, 79%) was obtained in the same way as in Example (1g) from the compound (169 mg) obtained in Example (1f) and 3,5-dimethoxy-phenyl isocyanate (105 mg). This methyl ester (203 mg) was hydrolyzed to obtain the title compound (182 mg, 92%) as a white solid.
$^1$H NMR (400 MHz, DMSO-d6): δ (ppm)=12.0 (1H, s), 8.64 (1H, s), 8.56 (1H, s), 7.32 (1H, s), 7.20 (1H, d, J=8.6 Hz), 7.08 (1H, d, J=8.6 Hz), 6.67 (2H, d, J=1.9 Hz), 6.14 (1H, dd, J=2.4 and 2.4 Hz), 4.48-4.46 (3H, m), 3.71 (6H, s), 3.56 (2H, t, J=5.4 Hz), 2.75 (2H, t, J=5.5 Hz), 2.12 (2H, d, J=7.1 Hz), 1.95-1.91 (2H, m), 1.77-1.73 (2H, m), 1.68-1.62 (1H, m), 1.39-1.29 (2H, m), 1.12-1.02 (2H, m)
MS (ESI) m/z: 512 $(M + H)^+$.
(Example 40)

trans-6-[3-(3,4-dichloro-phenyl)-ureido]-3,4-dihydro-1H-isoquinoline-2-carboxylic acid 4-carboxymethyl-cyclohexyl ester

[Formula 40]

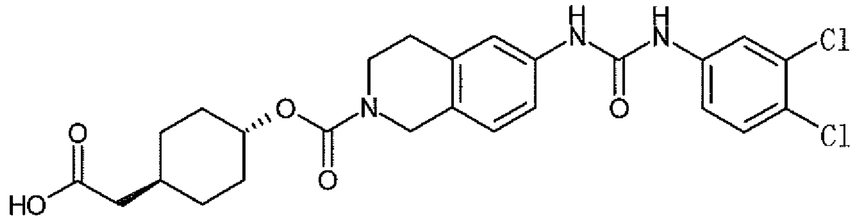

Methyl ester (236 mg, 91%) was obtained in the same way as in Example (1g) from the compound (169 mg) obtained in Example (1f) and 3,4-dichloro-phenyl isocyanate (111 mg). This methyl ester (236 mg) was hydrolyzed to obtain the title compound (212 mg, 92%) as a yellow solid.
$^{1}$H NMR (400 MHz, DMSO-d6): δ (ppm)=12.1 (1H, s), 9.06 (1H, s), 8.81 (1H, s), 7.89 (1H, d, J=2.3 Hz), 7.52 (1H, d, J=9.0 Hz), 7.32 (1H, dd, J=8.8 and 2.5 Hz), 7.32 (1H, s), 7.22 (1H, d, J=7.8 Hz), 7.10 (1H, d, J=8.6 Hz), 4.49-4.46 (3H, m), 3.57 (2H, t, J=6.1 Hz), 2.75 (2H, t, J=5.6 Hz), 2.12 (2H, d, J=7.1 Hz), 1.95-1.91 (2H, m), 1.77-1.73 (2H, m), 1.68-1.62 (1H, m), 1.39-1.29 (2H, m), 1.12-1.02 (2H, m)
MS (ESI) m/z: 521 $(M + H)^+$.
(Example 41)

trans-6-[3-(3-chloro-4-methoxy-phenyl)-ureido]-3,4-dihydro-1H-isoquinoline-2-carboxylic acid 4-carboxymethyl-cyclohexyl ester

[Formula 41]

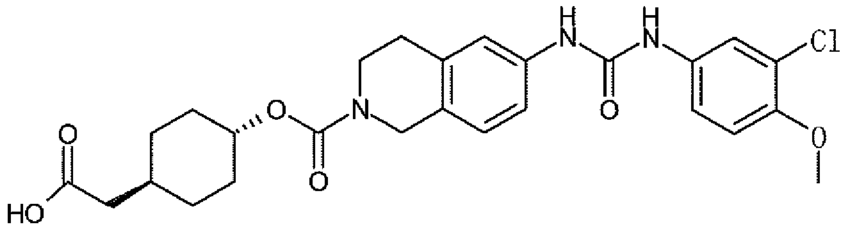

Methyl ester (181 mg, 75%) was obtained in the same way as in Example (1g) from the compound (158 mg) obtained in Example (1f) and 3-chloro-4-methoxy-phenyl isocyanate (101 mg). This methyl ester (181 mg) was hydrolyzed to obtain the title compound (153 mg, 87%) as a white solid.
$^{1}$H NMR (400 MHz, DMSO-d6): δ (ppm)=12.1 (1H, s), 8.63 (1H s), 8.60 (1H, s), 7.66 (1H, d, J=2.7 Hz), 7.31 (1H, s), 7.25 (1H, dd, J=9.0 and 2.7 Hz), 7.21 (1H, d, J=7.8 Hz), 7.08 (2H, d, J=9.0 Hz), 4.49-4.45 (3H, m), 3.81 (3H, s), 3.56 (2H, t, J=5.9 Hz), 2.75 (2H, t, J=5.9 Hz), 2.12 (2H, d, J=7.1 Hz), 1.95-1.91 (2H, m), 1.77-1.73 (2H, m), 1.68-1.62 (1H, m), 1.39-1.29 (2H, m), 1.12-1.02 (2H, m)
MS (ESI) m/z: 516 $(M + H)^+$.
(Example 42)

trans-6-[3-(2-fluoro-3-methoxy-phenyl)-ureido]-3,4-dihydro-1H-isoquinoline-2-carboxylic acid 4-carboxymethyl-cyclohexyl ester

[Formula 42]

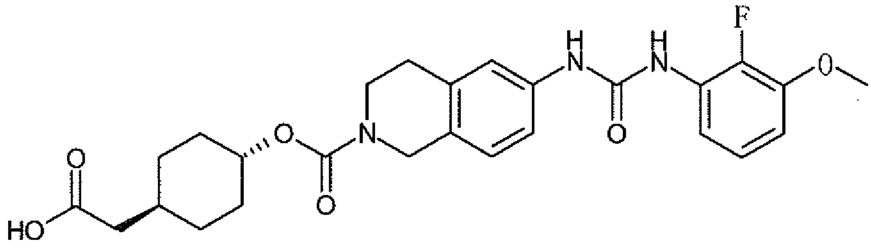

Methyl ester (266 mg, 75%) was obtained in the same way as in Example 27 from the compound (241 mg) obtained in Example (1f) and 2-fluoro-3-methoxy-benzoic acid (118 mg). This methyl ester (266 mg) was hydrolyzed to obtain the title compound (225 mg, 87%) as an off-white solid.
$^1$H NMR (400 MHz, DMSO-d6): δ (ppm)=12.0 (1H, s), 9.03 (1H, s), 8.51 (1H, d, J=2.4 Hz), 7.73 (1H, dd, J=7.0 and 7.0 Hz), 7.31 (1H, s), 7.21 (1H, d, J=8.6 Hz), 7.10 (1H, d, J=8.6 Hz), 7.04 (1H, dd, J=8.4 and 8.5 Hz), 6.80 (1H, dd, J=8.2 and 8.2 Hz), 4.51-4.46 (3H, m), 3.83 (3H, s), 3.57 (2H, t, J=5.9 Hz), 2.76 (2H, t, J=5.9 Hz), 2.12 (2H, d, J=6.7 Hz), 1.95-1.91 (2H, m), 1.76-1.74 (2H, m), 1.68-1.62 (1H, m), 1.39-1.29 (2H, m), 1.12-1.02 (2H, m)
MS (ESI) m/z: 500 (M + H)$^+$.
(Example 43)

trans-6-[3-(3,5-dichloro-phenyl)-ureido]-3,4-dihydro-1H-isoquinoline-2-carboxylic acid 4-carboxymethyl-cyclohexyl ester

[Formula 43]

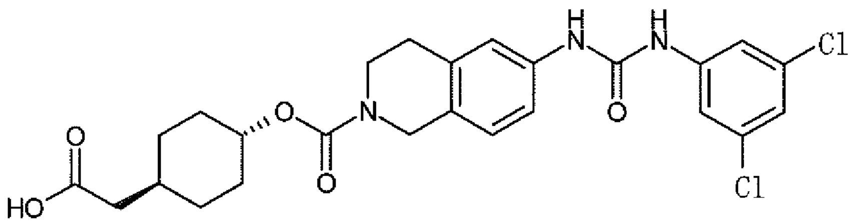

Methyl ester (268 mg, 95%) was obtained in the same way as in Example (1g) from the compound (184 mg) obtained in Example (1f) and 3,5-dichloro-phenyl isocyanate (120 mg). This methyl ester (268 mg) was hydrolyzed to obtain the title compound (234 mg, 90%) as a yellow solid.
$^1$H NMR (400 MHz, DMSO-d6): δ (ppm)=12.0 (1H, brs), 9.07 (1H, s), 8.84 (1H, s), 7.53 (2H, d, J=1.9 Hz), 7.32 (1H, s), 7.22 (1H, d, J=8.2 Hz), 7.16 (1H, dd, J=1.9 and 2.0 Hz), 7.10 (1H, d, J=8.6 Hz), 4.52-4.47 (3H, m), 3.57 (2H, t, J=5.8 Hz), 2.75 (2H, t, J=6.1 Hz), 2.12 (2H, d, J=6.7 Hz), 1.95-1.91 (2H, m), 1.77-1.73 (2H, m), 1.68-1.62 (1H, m), 1.39-1.29 (2H, m), 1.12-1.02 (2H, m)
MS (ESI) m/z: 521 (M + H)$^+$.
(Example 44)

trans-6-[3-(3,4-dimethoxy-phenyl)-ureido]-3,4-dihydro-1H-isoquinoline-2-carboxylic acid 4-carboxymethyl-cyclohexyl ester

[Formula 44]

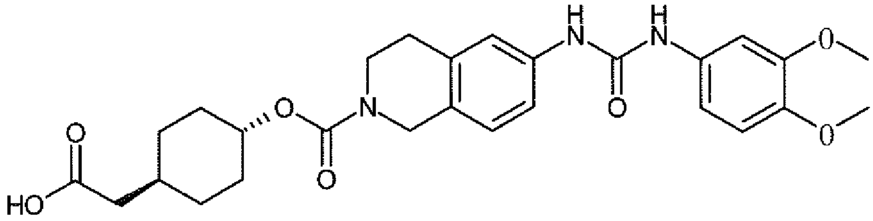

Methyl ester (377 mg, quantitative yield) was obtained in the same way as in Example (1g) from the compound (221 mg) obtained in Example (1f) and 3,4-dimethoxy-phenyl isocyanate (0.11 mL). This methyl ester (377 mg) was hydrolyzed to obtain the title compound (181 mg, 55%) as a white solid.
$^1$H NMR (400 MHz, DMSO-d6): δ (ppm)=12.0 (1H, s), 8.50 (1H, s), 8.47 (1H, s), 7.32 (1H, s), 7.22-7.18 (2H, m), 7.07 (1H, d, J=8.6 Hz), 6.86 (2H, s), 4.49-4.45 (3H, m), 3.74 (3H, s), 3.71 (3H, s), 3.56 (2H, t, J=5.8 Hz), 2.75 (2H, t, J=5.9 Hz), 2.12 (2H, d, J=7.1 Hz), 1.95-1.91 (2H, m), 1.77-1.74 (2H, m), 1.69-1.61 (1H, m), 1.39-1.29 (2H, m), 1.12-1.02 (2H, m)
MS (ESI) m/z: 512 (M + H)$^+$.
(Example 45)

trans-6-[3-(3-fluoro-4-methoxy-phenyl)-ureido]-3,4-dihydro-1H-isoquinoline-2-carboxylic acid 4-carboxymethyl-cyclohexyl ester

[Formula 45]

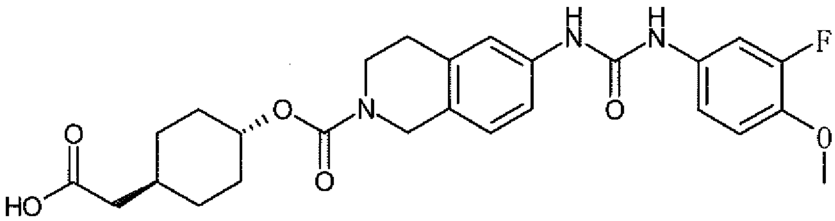

Methyl ester (303 mg, 96%) was obtained in the same way as in Example (1g) from the compound (213 mg) obtained in Example (1f) and 3-fluoro-4-methoxy-phenyl isocyanate (124 mg). This methyl ester (303 mg) was hydrolyzed to obtain the title compound (275 mg, 93%) as a white solid.
$^1$H NMR (400 MHz, DMSO-d6): δ (ppm)=12.1 (1H, s), 8.64 (1H, s), 8.59 (1H, s), 7.50-7.46 (2H, m), 7.31 (1H, s), 7.21 (1H, dd, J=9.6 and 1.8 Hz), 7.12-7.07 (2H, m), 4.49-4.46 (3H, m), 3.80 (3H, s), 3.57 (2H, t, J=6.0 Hz), 2.75 (2H, t, J=6.0 Hz), 2.12 (2H, d, J=7.0 Hz), 1.95-1.91 (2H, m), 1.78-1.74 (2H, m), 1.69-1.62 (1H, m), 1.39-1.30 (2H, m), 1.13-1.02 (2H, m)
MS (ESI) m/z: 500 (M + H)$^+$.
(Example 46)

trans-6-[3-(3-chloro-phenyl)-ureido]-3,4-dihydro-1H-isoquinoline-2-carboxylic acid 4-carboxymethyl-cyclohexyl ester

[Formula 46]

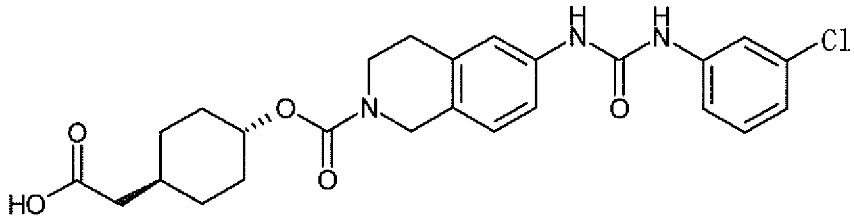

Methyl ester (5.16 g, 92%) was obtained in the same way as in Example (1g) from the compound (3.89 g) obtained in Example (1f) and 3-chloro-phenyl isocyanate (1.90 g). This methyl ester (5.16 g) was hydrolyzed to obtain the title compound (3.98 g, 79%) as a white solid.

$^1$H NMR (400 MHz, DMSO-d6): δ (ppm)=12.1 (1H, brs), 8.96 (1H, brs), 8.78 (1H, s), 7.72 (1H, s), 7.33 (1H, brs), 7.32-7.27 (1H, m), 7.24-7.21 (2H, m), 7.10 (1H, d, J=8.6 Hz), 7.01 (1H, d, J=7.4 Hz), 4.51-4.46 (3H, m), 3.57 (2H, t, J=5.9 Hz), 2.75 (2H, t, J=5.8 Hz), 2.12 (2H, d, J=7.0 Hz), 1.95-1.91 (2H, m), 1.76-1.74 (2H, m), 1.68-1.62 (1H, m), 1.39-1.29 (2H, m), 1.12-1.02 (2H, m)

MS (ESI) m/z: 486 $(M + H)^+$.

(Test Example 1)

(1) Preparation of DGAT1 enzyme

DGAT1 enzyme was prepared and stored according to the method described in MS2007/0249620.

(2) DGAT1 inhibitory activity test

A reaction solution having the following composition was incubated at room temperature (23°C) for 30 minutes: 175 mM Tris-HCl (pH 8.0), 8 mM $MgCl_2$, 1 mg/ml BSA, 0.3 mM 1,2-dioleoyl-sn-glycerol (10-fold concentration of EtOH solution, 10% added), 10 μM [$^{14}$C]-oleoyl-CoA (approximately 50 mCi/mmol), 0.5% Triton X-100, the DGAT1 enzyme (10 μg) obtained in Test Example 1(1), and a test compound or vehicle (DMSO/MeOH, 7:3 solution, 5% added), 50 μl in total. A reaction stop solution (70 μl) consisting of isopropanol/1-heptane/water (80:20:2, v/v/v) was added to the reaction solution and stirred. Subsequently, water (30 μl) and 1-heptane (100 μl) were added thereto and stirred. The 1-heptane layer (50 μl) was spotted onto a TLC plate and developed with a developing solvent consisting of 1-hexane/diethyl ether/acetic acid (85:15:1, v/v/v). The radioactivity of the triglyceride fraction was quantified using BAS2500 Bio Image Analyzer (FUJIFILM) and compared with the control. The inhibitory activity of the test compound was calculated according to the formula shown below. In this context, the radioactivity at no reaction (0 minute incubation) was used as background.

Inhibitory Rate = 100-[(Radioactivity of sample supplemented with test compound)-(Background)]/[(Radioactivity of control)-(Background)]×100

The compounds of Examples 1 to 46 exhibited a 50% or higher inhibitory rate at the test compound concentration of 0.05 μg/ml.

In this context, the DGAT inhibitory activity test is not limited to the method described above. For example, microsomes prepared from the small intestine, fat tissue, or liver of animals (e.g., rats or mice) may be used as the DGAT enzyme. Moreover, microsomes prepared from cultured cells (3T3-L1 fat cells, primarily cultured fat cells, Caco2 cells, HepG2 cells, etc.) or cultured cells highly expressing DGAT can also be used as the DGAT enzyme. Furthermore, flush plates (PerkinElmer) that require no extraction procedure can be used for efficiently evaluating a large number of

test compounds in a short time.

As is evident from these results, compounds of the present invention have excellent DGAT1 inhibitory bioactivity.

(Test Example 2)

The DGAT1 enzyme is important for the digestion and absorption of triglycerides, and the inhibition of DGAT1 in the small intestine suppresses the absorption of triglycerides. The in vivo activity of its DGAT1 inhibitory effect was evaluated with the suppressed absorption of triglycerides after fat loading as an index. Male C57BL/6N mice (7-12 weeks old, body weight: 17-25 g, Charles River Laboratories Japan Inc.) fasted overnight were assigned to Vehicle group 1, Vehicle group 2, and each test compound group, to which a vehicle (0.5% methylcellulose) or each test compound (1 to 10 mg/kg) suspended in vehicle was orally administered (5 mL/kg). After a given time, a lipoprotein lipase inhibitor (Pluronic-F127: Sigma-Aldrich Corp., 1 g/kg, 20% by weight dissolved in saline) was intraperitoneally administered (5 mL/kg) to each group. Immediately thereafter, distilled water for the Vehicle group 1 or a 20% fat-containing emulsion (Intralipid 20%: TERUMO CORP., Japan) for the Vehicle group 2 and the compound groups were orally administered thereto (0.2 mL/mouse). After a given time of 1 to 4 hours after administration, blood was collected from the tail vein or the right ventricle, and plasma was immediately separated and collected. Then, the triglyceride concentration in plasma was measured using a commercially available kit (Triglyceride E-Test Wako: Wako Pure Chemical Industries, Ltd.). In this method, the administration of the lipoprotein lipase inhibitor suppresses the hydrolysis of triglycerides in the blood such that the triglycerides are accumulated in the blood. These triglycerides are derived from two origins: exogenous triglycerides absorbed in the gastrointestinal tract and endogenous triglycerides released from the liver. The triglyceride absorption suppressive activity of each test compound was calculated based on the equation shown below by removing the influence of the endogenous triglycerides. In this context, each test compound was separately confirmed to have no influence on the concentration of the endogenous triglycerides.

Triglyceride absorption suppressive activity (%) = 100-[(Triglyceride concentration of each test compound group)-(Triglyceride concentration of Vehicle group 1)]/[(Triglyceride concentration of Vehicle group 2)-(Triglyceride concentration of Vehicle group 1)]xl00

The compounds of Examples 1 to 46 exhibited 60% or higher triglyceride absorption suppressive activity at a dose of 10 mg/kg or lower.

As is evident from these results, compounds of the present invention have excellent triglyceride absorption suppressive activity.

(Test Example 3)

Male C57BL/6N mice (7-12 weeks old, body weight: 17-25 g, Charles River Laboratories Japan Inc.) were individually housed and fed on a high-fat diet (fat content: 45 kcal%: Research Diets, Inc. D12451) for 1 week or longer for acclimatization. Based on the food intake during the period, the animals were equally assigned to experimental groups and fasted overnight. Then, a vehicle (0.5% methylcellulose) or each test compound (1 to 10 mg/kg) suspended in vehicle was orally administered (10 mL/kg) to each group. 30 minutes after administration, the animals were fed on a high-fat diet, and the food intake was measured 6 hours after initiation of feeding. The feeding suppressant activity of each test compound was calculated based on the equation shown below.

Feeding suppressant activity (%) = [(Food intake of Vehicle group)-(Food intake of each test compound group)]/[(Food intake of Vehicle group)]×100

The compound of Example 2 exhibited 70% feeding suppressant activity at a dose of 3 mg/kg.

As is evident from these results, compounds of the present invention have an excellent feeding suppressant effect.

In this context, the high-fat diet used as feed is not limited to the above-mentioned high-fat diet. For example, feed for rodents containing 45 to 60% triglycerides as calories can be used.

Formulation Example 1: Capsule

| | |
|---|---|
| Compound of Example 2 or 3 | 50 mg |
| Lactose | 128 mg |
| Corn starch | 70 mg |
| Magnesium stearate | 2 mg |
| | 250 mg |

A powder of this formulation is mixed and passed through a 60-mesh sieve. Then, this powder is charged into 250 mg of gelatin capsule to prepare a capsule.

Formulation Example 2: Tablet

| | |
|---|---|
| Compound of Example 2 or 3 | 50 mg |
| Lactose | 126 mg |
| Corn starch | 23 mg |
| Magnesium stearate | 1 mg |
| | 200 mg |

A powder of this formulation is mixed, granulated using a corn starch paste, then dried, and then compressed into a tablet (200 mg/tablet) using a tableting machine. This tablet can be sugar-coated according to need.

Industrial Applicability

A compound of the present invention or a pharmacologically acceptable salt thereof has an excellent DGAT inhibitory effect and feeding suppressant effect and is thus useful as a pharmaceutical agent.

## Claims

**1.** Trans-6-[3-(2,4-difluoro-phenyl)-ureido]-3,4-dihydro-1H-isoquinoline-2-carboxylic acid 4-carboxymethyl-cyclohexyl ester,

trans-6-[3-(2-chloro-phenyl)-ureidol-3,4-dihydro-1H-isoquinoline-2-carboxylic acid 4-carboxymethyl-cyclohexyl ester,
trans-6-[3-(2,3-difluoro-phenyl)-ureido]-3,4-dihydro-1H-isoquinoline-2-carboxylic acid 4-carboxymethyl-cyclohexyl ester,
trans-6-[3-(2,5-difluoro-phenyl)-ureido]-3,4-dihydro-1H-isoquinoline-2-carboxylic acid 4-carboxymethyl-cyclohexyl ester,
trans-6-[3-(2,6-difluoro-phenyl)-ureido]-3,4-dihydro-1H-isoquinoline-2-carboxylic acid 4-carboxymethyl-cyclohexyl ester,
trans-6-[3-(2-fluoro-3-methyl-phenyl)-ureido]-3,4-dihydro-1H-isoquinoline-2-carboxylic acid 4-carboxymethyl-cyclohexyl ester,
trans-6-[3-(2-fluoro-5-methyl-phenyl)-ureido]-3,9-dihydro-1H-isoquinoline-2-carboxylic acid 4-carboxymethyl-cyclohexyl ester,
trans-6-[3-(2-fluoro-4-methyl-phenyl)-ureido]-3,4-dihydro-1H-isoquinoline-2-carboxylic acid 4-carboxymethyl-cyclohexyl ester,
trans-6-[3-(2-fluoro-6-methyl-phenyl)-ureido]-3,4-dihydro-1H-isoquinoline-2-carboxylic acid 4-carboxymethyl-cyclohexyl ester,
trans-6-[3-(3-chloro-2-fluoro-phenyl)-ureido]-3,4-dihydro-1H-isoquinoline-2-carboxylic acid 4-carboxymethyl-cyclohexyl ester,

trans-6-[3-(4-chloro-2-fluoro-phenyl)-ureido]-3,4-dihydro-1H-isoquinoline-2-carboxylic acid 4-carboxymethyl-cyclohexyl ester,
trans-6-[3-(5-chloro-2-fluoro-phenyl)-ureido]-3,4-dihydro-1H-isoquinoline-2-carboxylic acid 4-carboxymethyl-cyclohexyl ester,
trans-6-[3-(2-chloro-6-fluoro-phenyl)-ureido]-3,4-dihydro-1H-isoquinoline-2-carboxylic acid 4-carboxymethyl-cyclohexyl ester,
trans-6-[3-(2,3,4-trifluoro-phenyl)-ureido]-3,4-dihydro-1H-isoquinoline-2-carboxylic acid 4-carboxymethyl-cyclohexyl ester,
trans-6-[3-(2,4,6-trifluoro-phenyl)-ureido]-3,4-dihydro-1H-isoquinoline-2-carboxylic acid 4-carboxymethyl-cyclohexyl ester,
trans-6-[3-(2-fluoro-4-trifluoromethyl-phenyl)-ureido]-3,4-dihydro-1H-isoquinoline-2-carboxylic acid 4-carboxymethyl-cyclohexyl ester,
trans-6-[3-(2-trifluoromethyl-phenyl)-ureido]-3,4-dihydro-1H-isoquinoline-2-carboxylic acid 4-carboxymethyl-cyclohexyl ester,
trans-6-[3-(4-fluoro-2-trifluoromethyl-phenyl)-ureido]-3,4-dihydro-1H-isoquinoline-2-carboxylic acid 4-carboxymethyl-cyclohexyl ester,
trans-6-[3-(4-methoxy-2-methyl-phenyl)-ureido]-3,4-dihydro-1H-isoquinoline-2-carboxylic acid 4-carboxymethyl-cyclohexyl ester,
trans-6-[3-(2-chloro-4-methyl-phenyl)-ureido]-3,4-dzhydro-1H-isoquinoline-2-carboxylic acid 4-carboxymethyl-cyclohexyl ester,
trans-6-[3-(2-fluoro-3-trifluoromethyl-phenyl)-ureido]-3,4-dihydro-1H-isoquinoline-2-carboxylic acid 4-carboxymethyl-cyclohexyl ester,
trans-6-[3-(2-fluoro-5-triflucromethyl-phenyl)-ureido]-3,4-dihydro-1H-isoquinoline-2-carboxylic acid 4-carboxymethyl-cyclohexyl ester,
trans-6-[3-(2,4-dimethyl-phenyl)-ureido]-3,4-dihydro-1H-isoquinoline-2-carboxylic acid 4-carboxymethyl-cyclohexyl ester,
trans-5-[3-(2,4-dichloro-phenyl)-ureido]-3,4-dihydro-1H-isoquinoline-2-carboxylic acid 4-carboxymethyl-cyclohexyl ester,
trans-6-[3-(2-fluoro-6-trifluoromethyl-phenyl)-ureido]-3,4-dihydro-1H-isoquinoline-2-carboxylic acid 4-carboxymethyl-cyclohexyl ester,
trans-6-[3-(4-chloro-2-methyl-phenyl)-ureido]-3,4-dihydro-1H-isoquinoline-2-carboxylic acid 4-carboxymethyl-cyclohexyl ester,
trans-6-[3-(2-chloro-3-methyl-phenyl)-ureido]-3,4-dihydro-1H-isoquinoline-2-carboxylic acid 4-carboxymethyl-cyclohexyl ester,
trans-6-[3-(4-chloro-2-trifluoromethyl-phenyl)-ureido]-3,4-dihydro-1H-isoquinoline-2-carboxylic acid 4-carboxymethyl-cyclohexyl ester,
trans-6-[3-(2-chloro-4-trifluoromethyl-phenyl)-ureido]-3,4-dihydro-1H-isoquinoline-2-carboxylic acid 4-carboxymethyl-cyclohexyl ester,
trans-6-[3-(3-fluoro-4-methyl-phenyl)-ureido]-3,4-dihydro-1H-isoquinoline-2-carboxylic acid 4-carboxymethyl-cyclohexyl ester,
trans-6-[3-(3,5-difluoro-phenyl)-ureido]-3,4-dihydro-1H-isoquinoline-2-carboxylic acid 4-carboxymethyl-cyclohexyl ester,
trans-6-[3-(3-chloro-4-fluoro-phenyl)-ureido]-3,4-dihydro-1H-isoquinoline-2-carboxylic acid 4-carboxymethyl-cyclohexyl ester,
trans-6-[3-(3,5-dimethyl-phenyl)-ureido]-3,4-dihydro-1H-isoquinoline-2-carboxylic acid 4-carboxymethyl-cyclohexyl ester,
trans-6-[3-(3-chloro-4-methyl-phenyl)-ureido]-3,4-dihydro-1H-isoquinoline-2-carboxylic acid 4-carboxymethyl-cyclohexyl ester,
trans-6-[3-(2,3-dimethyl-phenyl)-ureido]-3,4-dihydro-1H-isoquinoline-2-carboxylic acid 4-carboxymethyl-cyclohexyl ester,
trans-6-[3-(3-chloro-2-methyl-phenyl)-ureido]-3,4-dihydro-1H-isoquinoline-2-carboxylic acid 4-carboxymethyl-cyclohexyl ester,
trans-6-[3-(2,3-dichloro-phenyl)-ureido]-3,4-dihydro-1H-isoquinoline-2-carboxylic acid 4-carboxymethyl-cyclohexyl ester,
trans-6-[3-(4-fluoro-3-methyl-phenyl)-ureido]-3,4-dihydro-1H-isoquinoline-2-carboxylic acid 4-carboxymethyl-cyclohexyl ester,
trans-6-[3-(3,5-dimethoxy-phenyl)-ureido]-3,4-dihydro-1H-isoquinoline-2-carboxylic acid 4-carboxymethyl-cyclohexyl ester,

trans-6-[3-(3,4-dichloro-phenyl)-ureido]-3,4-dihydro-1H-isoquinoline-2-carboxylic acid 4-carboxymethyl-cyclohexyl ester,
trans-6-[3-(3-chloro-4-methoxy-phenyl)-ureido]-3,4-dihydro-1H-isoquinoline-2-carboxylic acid 4-carboxymethyl-cyclohexyl ester,
trans-6-[3-(2-fluoro-3-methoxy-phenyl)-ureido]-3,4-dihydro-1H-isoquinoline-2-carboxylic acid 4-carboxymethyl-cyclohexyl ester,
trans-6-[3-(3,5-dichloro-phenyl)-ureido]-3,4-dihydro-1H-isoquinoline-2-carboxylic acid 4-carboxymethyl-cyclohexyl ester,
trans-6-[3-(3,4-dimethoxy-phenyl)-ureido]-3,4-dihydro-1H-isoquinoline-2-carboxylic acid 4-carboxymethyl-cyclohexyl ester,
trans-6-[3-(3-fluoro-4-methoxy-phenyl)-ureido]-3,4-dihydro-1H-isoquinoline-2-carboxylic acid 4-carboxymethyl-cyclohexyl ester, or
trans-6-[3-(3-chloro-phenyl)-ureido]-3,4-dihydro-1H-isoquinoline-2-carboxylic acid 4-carboxymethyl-cyclohexyl ester,

or a pharmacologically acceptable salt thereof.

**2.** An acyl-coenzyme A: diacylglycerol acyltransferase inhibitor comprising as an active ingredient thereof a compound or pharmacologically acceptable salt thereof according to claim 1.

**3.** A feeding suppressant and/or an anorectic comprising as an active ingredient thereof a compound or pharmacologically acceptable salt thereof according to claim 1.

**4.** A pharmaceutical composition comprising as an active ingredient thereof a compound or pharmacologically acceptable salt thereof according to claim 1.

**5.** The pharmaceutical composition according to claim 4, wherein the pharmaceutical composition has an acyl-coenzyme A: diacylglycerol acyltransferase inhibitory effect.

**6.** The pharmaceutical composition according to claim 4, wherein the pharmaceutical composition has a feeding suppressant effect and/or an anorectic effect.

**7.** The pharmaceutical composition according to claim 4, wherein the pharmaceutical composition is intended for the treatment and/or prevention of a disease whose symptoms are treated, improved, alleviated and/or prevented by inhibiting acyl-coenzyme A: diacylglycerol acyltransferase such that triglyceride synthesis is inhibited, resulting in suppressed absorption of triglyceride.

**8.** The pharmaceutical composition according to claim 4, wherein the pharmaceutical composition is intended for the treatment and/or prevention of a disease whose symptoms are treated, improved, alleviated and/or prevented by inhibiting acyl-coenzyme A: diacylglycerol acyltransferase such that triglyceride synthesis is inhibited.

**9.** The pharmaceutical composition according to claim 4, wherein the pharmaceutical composition is intended for the treatment and/or prevention of adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, nonalcoholic steatohepatitis, polycystic ovary syndrome, arteriosclerosis, atherosclerosis, diabetic atherosclerosis, ischemic heart disease, or bulimia.

**10.** The pharmaceutical composition according to claim 4, wherein the pharmaceutical composition is intended for the treatment and/or prevention of adiposity or obesity.

**11.** The pharmaceutical composition according to claim 4, wherein the pharmaceutical composition is intended for the treatment and/or prevention of diabetes.

**12.** Use of a compound or pharmacologically acceptable salt thereof according to claim 1, for producing a pharmaceutical composition.

**13.** The use according to claim 12, wherein the pharmaceutical composition is a composition intended for the treatment

and/or prevention of adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, nonalcoholic steatohepatitis, polycystic ovary syndrome, arteriosclerosis, atherosclerosis, diabetic atherosclerosis, ischemic heart disease, or bulimia.

**14.** A method for treating and/or preventing a disease, comprising administering a pharmacologically effective amount of a compound or pharmacologically acceptable salt thereof according to claim 1 to a warm-blooded animal.

**15.** The method according to claim 14, wherein the disease is adiposity, obesity, hyperlipidemia, hypertriglyceridemia, lipidosis, insulin resistance syndrome, impaired glucose tolerance, diabetes, diabetic complications (including diabetic peripheral neuropathy, diabetic nephropathy, diabetic retinopathy, and diabetic macroangiopathy), cataract, gestational diabetes mellitus, nonalcoholic steatohepatitis, polycystic ovary syndrome, arteriosclerosis, atherosclerosis, diabetic atherosclerosis, ischemic heart disease, or bulimia.

**16.** The method according to claim 14 or 15, wherein the warm-blooded animal is a human.

## INTERNATIONAL SEARCH REPORT

International application No.
PCT/JP2010/064550

**A. CLASSIFICATION OF SUBJECT MATTER**
*C07D217/06*(2006.01)i, *A61K31/472*(2006.01)i, *A61P1/16*(2006.01)i, *A61P3/04*(2006.01)i, *A61P3/06*(2006.01)i, *A61P3/10*(2006.01)i, *A61P9/10*(2006.01)i, *A61P15/08*(2006.01)i, *A61P27/02*(2006.01)i, *A61P43/00*(2006.01)i
According to International Patent Classification (IPC) or to both national classification and IPC

**B. FIELDS SEARCHED**
Minimum documentation searched (classification system followed by classification symbols)
C07D217/06, A61K31/472, A61P1/16, A61P3/04, A61P3/06, A61P3/10, A61P9/10, A61P15/08, A61P27/02, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus/REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| X | WO 2007/074753 A1 (Daiichi Sankyo Co., Ltd.), 05 July 2007 (05.07.2007), claims; page 63, compounds 3-66 to 3-70; test examples (Family: none) | 1-13 |
| X | JP 2006-45209 A (Sankyo Co., Ltd.), 16 February 2006 (16.02.2006), claims; page 59, compounds 3-66 to 3-70; test examples & US 2007/0249620 A1 & EP 1764360 A1 & WO 2006/004200 A1 & CA 2572874 A | 1-13 |
| P,X | WO 2009/119534 A1 (DAIICHI SANKYO CO., LTD.), 01 October 2009 (01.10.2009), entire text (Family: none) | 1-13 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 September, 2010 (28.09.10) | 12 October, 2010 (12.10.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/064550

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 14-16
because they relate to subject matter not required to be searched by this Authority, namely:
Claims 14-16 involve "methods for treatment of the human body or animal body by surgery or therapy".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20070249620 A
- WO 2007074753 A


### Non-patent literature cited in the description


- **Eiji Itagaki.** STEP series, Metabolism, Endocrinology. KAIBASHOBO, LTD, 1998, 105
- **Zimmet, P. et al.** *Nature,* 2001, vol. 414, 782-787
- **Engstrom, R. G. et al.** *Arch. Intern. Pharmacodyn.,* 1975, vol. 214, 308-321
- **Bray, G. A. et al.** *Obes. Res.,* 1996, vol. 4, 263-270
- **Davidson, M. H. et al.** *The Journal of the American Medical Association,* 1999, vol. 281, 235-242
- **Coleman, R. ; Bell, R.** *J. Biol. Chem.,* 1976, vol. 251, 4537-4543
- **Coleman, R.** *Methods in Enzymology,* 1992, vol. 209, 98-104
- **Lehner, R. ; Kuksis, A.** *Prog. Lipid Res.,* 1996, vol. 35, 169-201
- **R. Bell.** *Ann. Rev. Biochem.,* 1980, vol. 49, 459-487
- **Cases, S. et al.** *Proc. Natl. Acad. Sci. USA.,* 1998, vol. 95, 13018-13023
- **Cases, S. et al.** *J. Biol. Chem.,* 2001, vol. 276, 38870-38876
- **Coleman, R. A. ; Lee, D. P.** *Progress in Lipid Research,* 2004, vol. 43, 134-176
- **Smith, S. J. et al.** *Nat. Genet.,* 2000, vol. 25, 87-90
- **Chen, H. C.** *J. Clin. Invest.,* 2002, vol. 109, 1049-1055
- **Buhman, K. K.** *J. Biol. Chem.,* 2002, vol. 277, 25474-25479
- **Gaziano, J. et al.** *Circulation,* 1997, vol. 96, 2520-2525
- **Yamaguchi, K. et al.** *Hepatology,* 2008, vol. 47, 625-635
- **Strader, A. D. et al.** *Gastroenterology,* 2005, vol. 128, 175-191
- **Campfield, L. A. et al.** *Science,* 1995, vol. 269, 546-549
- *J. Med. Chem.,* 1996, vol. 39, 1084
- *J. Org. Chem.,* 1998, vol. 63, 4116